# EUROPEAN PATENT APPLICATION

(11) **EP 4 124 662 A1**
(43) Date of publication of application: **01.02.2023**
(21) Application number: 21187791.5
(22) Date of filing: 26.07.2021
(51) Int. Cl.: C12Q 1/6886

(54) **PD-L1 MIRNAS FOR DISEASE PROGNOSIS**

(71) Applicant: Martin-Luther-Universität Halle-Wittenberg, 06108 Halle (Saale) (DE)
(72) Inventor: Seliger, Barbara, 06114 Halle (DE); Vaxevanis, Christoforos, 06108 Halle (DE); Friedrich, Michael, 35037 Marburg (DE)
(74) Representative: Grünecker Patent- und Rechtsanwälte PartG mbB

(57) **Abstract**

The present invention relates to methods for prognosing a cancer that expresses PD-L1 on the surface of tumor cells (PD-L1-positive cancer) in a patient, uses of miRNAs and kits.

## Description

### TECHNICAL FIELD

The present disclosure generally relates to a method for prognosing a cancer that expresses programmed death ligand 1 (PD-L1, CD274) on the surface of tumor cells (PD-L1-positive cancer) in a patient, use of miRNAs, in particular as biomarkers for prognosing PD-L1-positive cancer and kits for prognosing PD-L1 positive cancer.

### BACKGROUND OF THE INVENTION

Programmed death ligand 1 (PD-L1), also known as cluster of differentiation 274 (CD274) or B7-H1, is a type 1 transmembrane protein, which interacts with a T cell inhibitory receptor named programmed cell death protein-1 (PD1). The interaction between PD1 and PD-L1 on the cell surface of T cells promotes T cell tolerance, minimizes autoimmune mediated inflammation, but also results in an immune escape of tumor cells. PD-L1 is a checkpoint protein, which is expressed by various T cells and antigen presenting cells, but is also over-expressed in different cancer cells. Expression by cancer cells results in a reduced elimination of tumor cells by CD8+ cytotoxic T lymphocytes (CTL), thereby promoting tumor progression. Indeed, increased levels of PD-L1 expression have been associated with disease progression in cancer, high risk of cancer mortality as well as a poor patient outcome. Targeted therapies blocking the interaction between PD-L1 and PD-1 are a promising therapeutic option for various cancer types and already approved for the treatment of e.g. melanoma, non-small cell lung cancer (NSCLC), renal cell carcinoma (RCC), bladder cancer and head and neck squamous cell carcinoma (HNSCC).

Despite the promising results, the objective response rate of patients with various cancer types are still low. This might be due to the high individual variation of PD-L1 expression mediated by cellular and soluble components of the tumor microenvironment (TME) (Kim, J.M. and D.S. Chen, Ann Oncol, 2016. 27(8): p. 1492-504). Furthermore, it has been demonstrated that poor responders to immune checkpoint inhibitor (CPI) therapy express low PD-L1 levels and have a reduced T cell infiltration. Recently, a complex regulation of PD-L1 expression was reported including genomic alterations, transcriptional and epige-netic control, mRNA and protein stability, post-transcriptional regulation as well as inflammatory and oncogenic signaling.

Thus, there is a need for new diagnostic methods that allow prognosing PD-L1 positive cancer.

### SUMMARY OF THE INVENTION

In a first aspect, provided is a method for prognosing a cancer that expresses PD-L1 on the surface of tumor cells (PD-L1-positive cancer) in a patient, the method comprising the steps of: a) measuring in a sample from the patient the tumor expression level of at least one miRNA selected from the group consisting of the miRNAs according to SEQ ID NO: 1 to 60; b) comparing the tumor expression level determined in step a) with the tumor expression level of the same at least one miRNA in a reference patient group suffering from the same cancer, to determine a relative tumor expression level of the at least one miRNA; and c) determining a prognosis for the cancer based on the outcome of step b), wherein a high relative tumor expression level of the at least one miRNA relative to the expression of the at least one miRNA in the reference group contributes to a poor prognosis.

In an embodiment, the tumor expression level of one, two, three, four or five of the miRNAs selected from the group consisting of miR-29, miR-155, miR-181b, miR-186 and miR-17 is determined. Preferably, the tumor expression level of miR-29, miR-155, miR-181b, miR-186 and miR-17 is determined.

In an embodiment, the method further comprises the step of establishing a cell culture of a cell line that expresses PD-L1 on its surface and the step of measuring in vitro the reduction of PD-L1 surface expression of the cell line that expresses PD-L1 on its surface upon administration of the at least one miRNA or a miRNA mimic thereof; and wherein step c) is further based on the outcome of the step of the measuring in vitro the reduction of PD-L1 surface expression.

In an embodiment, the method further comprises a step of measuring in a sample from the patient PD-L1 surface expression on the tumor cells; and wherein step c) is further based on the outcome of the step of measuring in a sample from the patient PD-L1 surface expression.

In an embodiment, the method further comprises a step of measuring in a sample from the patient CD8+ T cell infiltration in the tumor tissue; and wherein step c) is further based on the outcome of the further step of measuring in a sample from the patient CD8+ T cell infiltration in a tumor tissue of the PD-L1-positive cancer.

In an embodiment, the cancer is selected from the group consisting of skin cancer, preferably melanoma, non-small cell lung cancer (NSCLC), Hodgkin's lymphoma, bladder cancer, renal cell carcinoma (RCC), head and neck squamous cell carcinoma (HNSCC), breast cancer, Merkel cell carcinoma, hepatocellular carcinoma (HCC) and gastric cancer (GC).

In an embodiment, a numerical value (miRNA worth) is attributed to each selected miRNA, wherein the miRNA worth corresponds to the degree of reduction that the miRNA or a miRNA mimic thereof causes in PD-L1 surface expression upon administration of the miRNA or a miRNA mimic thereof in cell culture to a PD-L1-positive cancer cell line, wherein the greater the reduction in PD-L1 surface expression upon administration of the miRNA or a miRNA mimic thereof, the greater the miRNA worth, wherein the miRNA worth is a positive number.

In an embodiment, a numerical value (expression multiplier) is attributed to each selected miRNA, wherein the expression multiplier corresponds to the tumor expression level of the miRNA as compared to the tumor expression level of the same miRNA in a group of patients suffering from the same cancer, wherein the greater the expression of the miRNA in the patient relative to the group of patients, the greater the expression multiplier, wherein the expression multiplier is a positive number or zero.

In an embodiment, the method further comprises calculating a numerical individual miRNA score for each selected miRNA that is the product of the miRNA worth for the selected miRNA and the expression multiplier (miRNA worth*expression multiplier) for the same selected miRNA.

In an embodiment, the method further comprises calculating a numerical PDM score, which PDM score is the sum of individual miRNA scores, by addition of each individual miRNA score for each selected miRNA, wherein the greater the PDM score, the poorer the prognosis.

In an embodiment, the miRNA worth for miR-29 is 1*a, the miRNA worth for miR-155 is 3*a, the miRNA worth for miR-181b is 2*a, the miRNA worth for miR-186 is 3*a and the miRNA worth for miR-17 is 3*a, wherein a is a factor (e.g. 1*a means 1 multiplied by a) and a positive number, preferably a equals 1.

In a further aspect, provided is a use of at least one miRNA selected from the group consisting of the miRNAs according to SEQ ID NO: 1 to 60 for prognosing PD-L1 positive cancer, in particular as a biomarker for prognosing PD-L1 positive cancer.

In an embodiment, one, two, three, four or five miRNAs selected from the group consisting of miR-29, miR-155, miR-181b, miR-186 and miR-17 are used for prognosing PD-L1 positive cancer.

In a further aspect, provided is a kit for prognosing PD-L1 positive cancer, the kit comprising polymerase chain reaction primers capable of or a microarray capable of detecting the expression of at least one miRNA selected from the group consisting of the miRNAs according to SEQ ID NO: 1 to 60.

In an embodiment, the polymerase chain reaction primers or the microarray are capable of detecting the expression of one, two, three, four or five miRNAs selected from the group consisting of miR-29, miR-155, miR-181b, miR-186 and miR-17.

The present kits, uses and methods are generally contemplated for in vitro use.

### BRIEF DESCRIPTION OF DRAWINGS

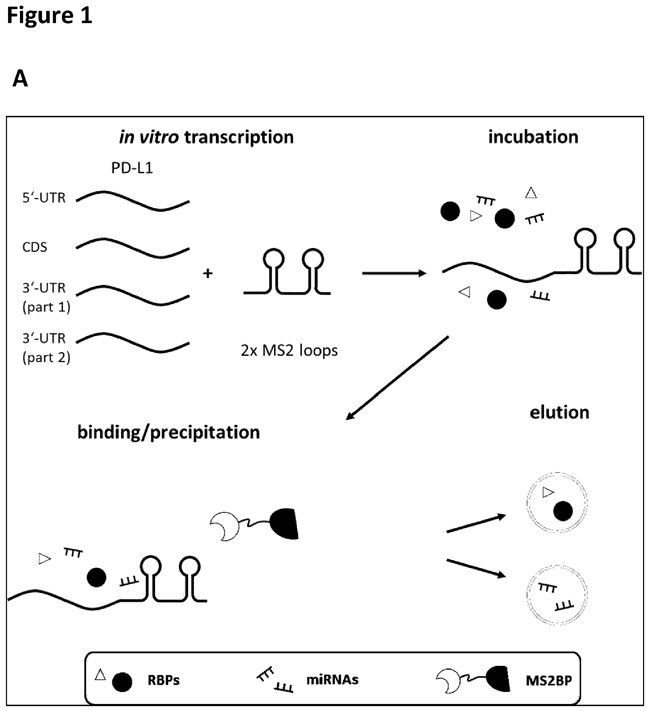

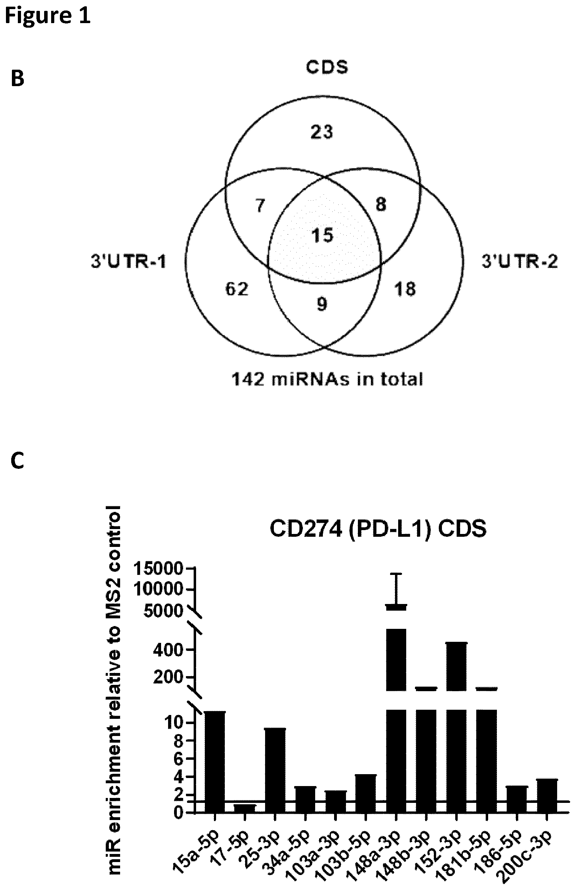

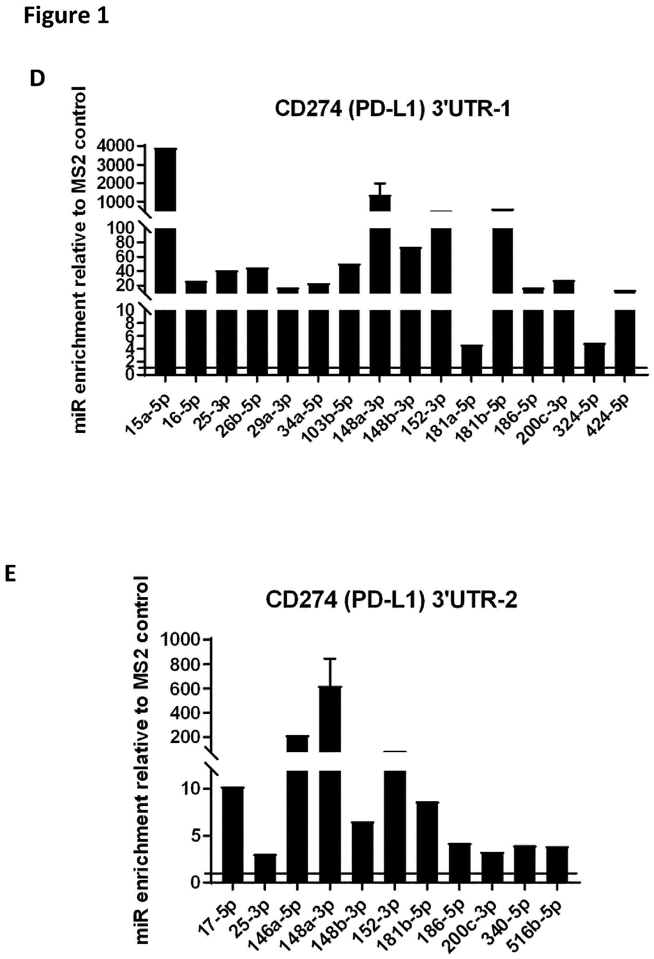

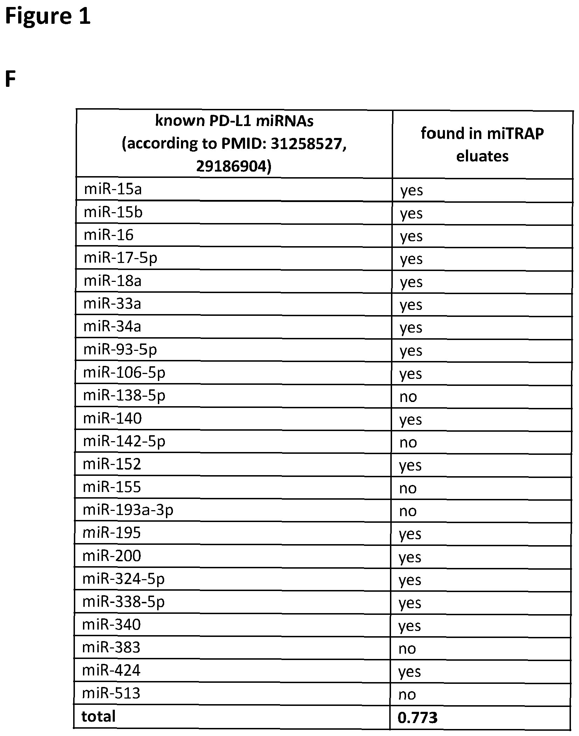

**Figure 1****.** Identification of PD-L1 targeting miRs. **A**) Graphical representation of the miTRAP method followed for the identification of PD-L1 specific miRNAs. **B**) Venn diagram of miRNAs identified relative to their binding to the three different regions of PD-L1 mRNA, represented as enrichment compared to the MS2 control. **C-E**) Validation of selected candidates by qPCR. The relative expression of the target miRNAs is depicted in comparison to the enrichment found in the MS2 control, which is set to 1 (black line). F) Known miRNAs found in miTRAP eluates.

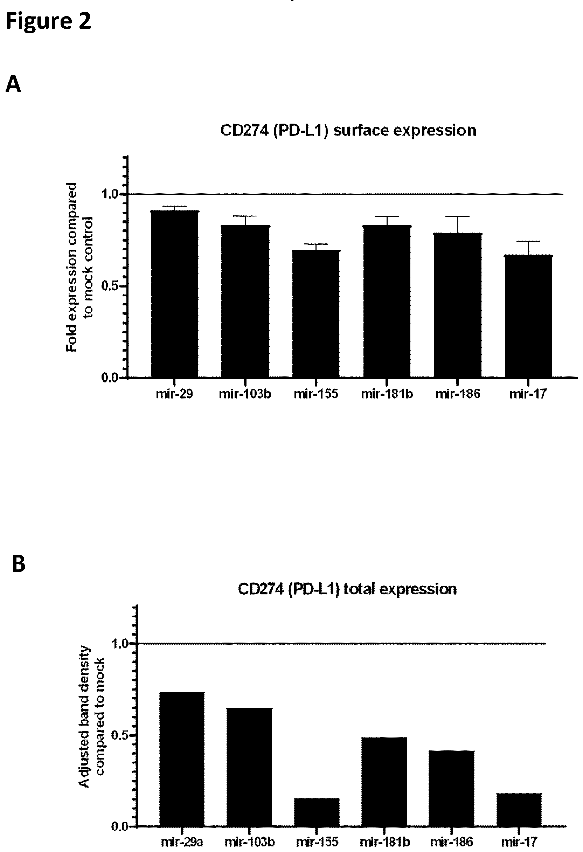

**Figure 2****.** PD-L1 expression and miRNA mimics. The relative surface expression (A) and total expression (B) of PD-L1 after transfection with miRNA mimics when compared to the mock control (horizontal line), as determined by flow cytometry and Western blot analysis.

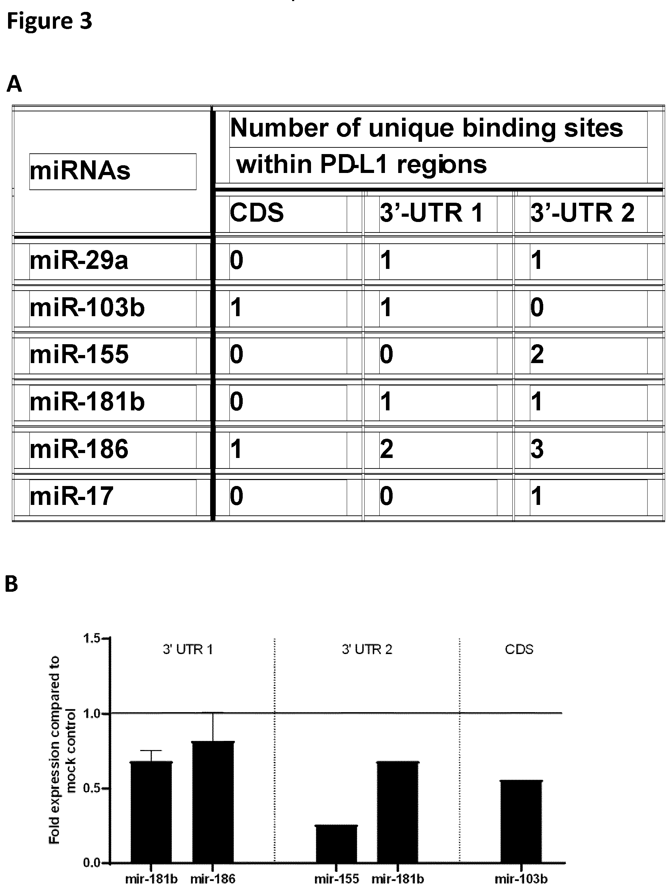

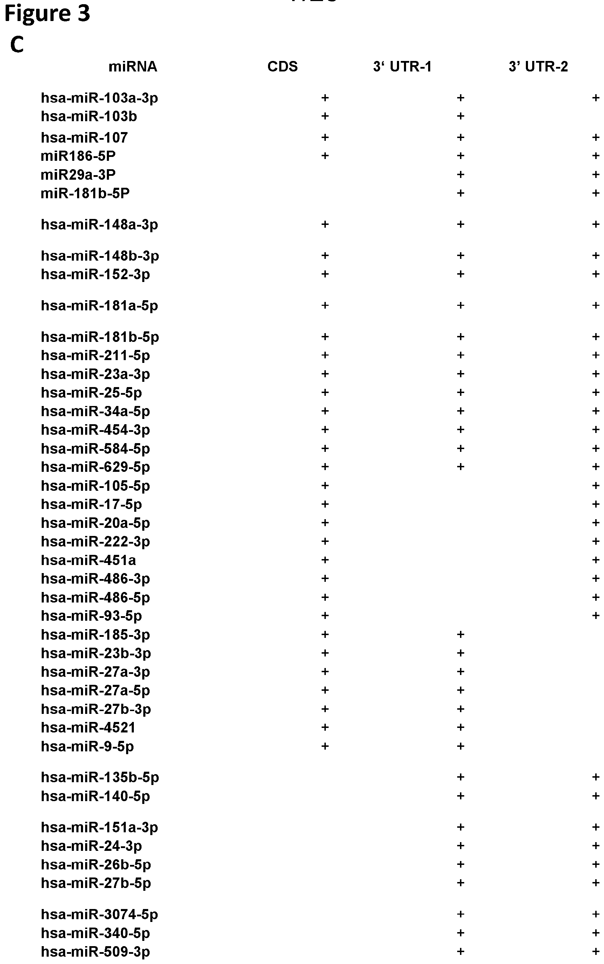

**Figure 3**. **A)** Number of putative binding sites of miRNAs in 3 different regions of PD-L1 mRNA. Binding sites were predicted through Target Scan 7.2, a miRNA prediction tool. B) Relative luminescence levels after transfection with miRNA mimics compared to the mock control (horizontal line), as assessed by dual luciferase assay. C) Location of the binding site of the miRNAs in the PD-L1 regions CDS, 3'UTR-1 and 3'UTR-2.

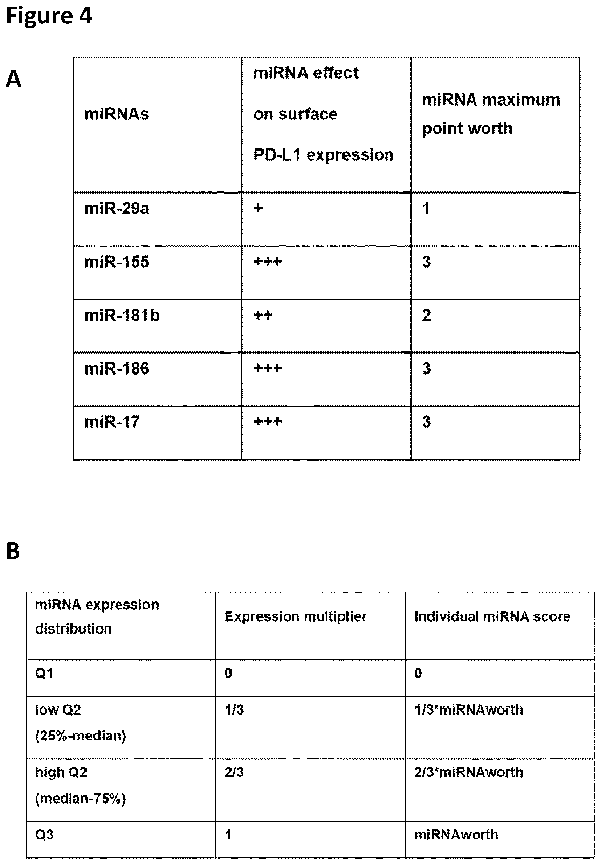

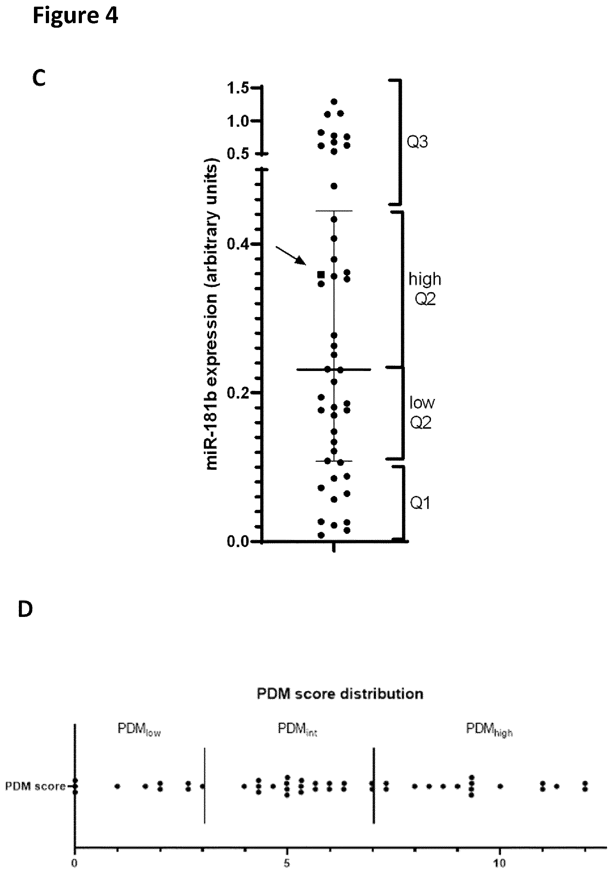

**Figure 4****.** Construction of the PDM score. **A**) Table of the maximum miRNA point worth (miRNA worth) for each of the individual miRNAs, based on the amount of PD-L1 surface reduction upon overexpression in mel1359 cells. Each "+"represents a 10 % reduction and corresponds to 1 point. **B**) Table determining the expression multiplier depending on the position within the quartiles after the total frequency distribution calculation among the patients for each miRNA. **C**) Total frequency distribution of all investigated patients. As shown in the graph, a patient with miR-181b expression within the 3rd quartile (arrow, between the median and 75 %) was awarded 2/3 of the miR-181b worth, adding a total of 1.67 points to the sum. **D**) PDM score and PDM score distribution of the total patients; A Patient's PDM score equals ("=") the sum of all individual miRNA scores (see table in (**B**)); In this case, the PDM score for miRNAs miR-29a, miR-155, miR181b, miR186 and miR-17 ranges from 0 to 12 (PDM scoreₘᵢₙ = 0, PDM scoreₘₐₓ = 12). The PDM score can be grouped into PDM_{low} (0< PDM_{low}≤3), PDMᵢₙₜ (3<PDMᵢₙₜ≤7) and PDM_{high} (7<PDM_{high}≤12) as indicated.

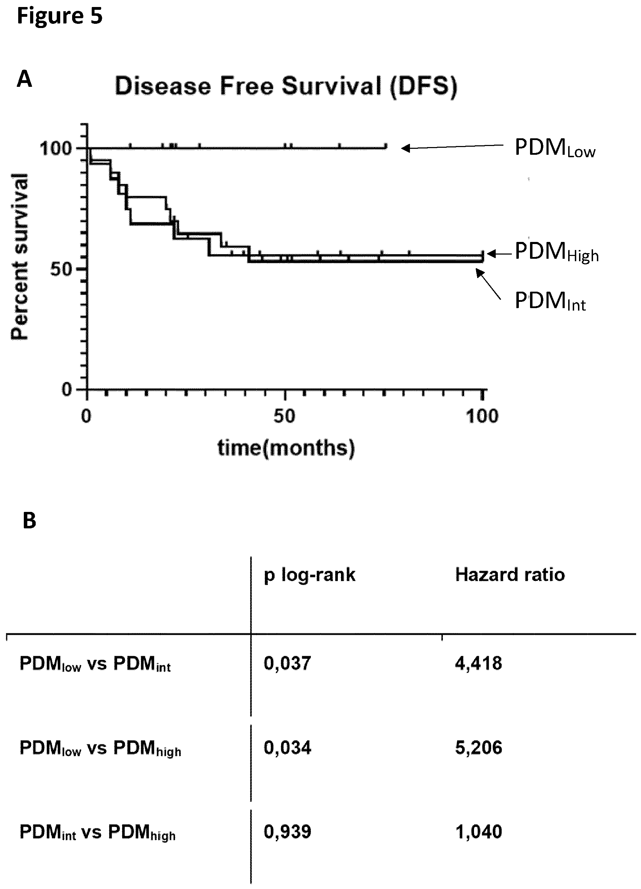

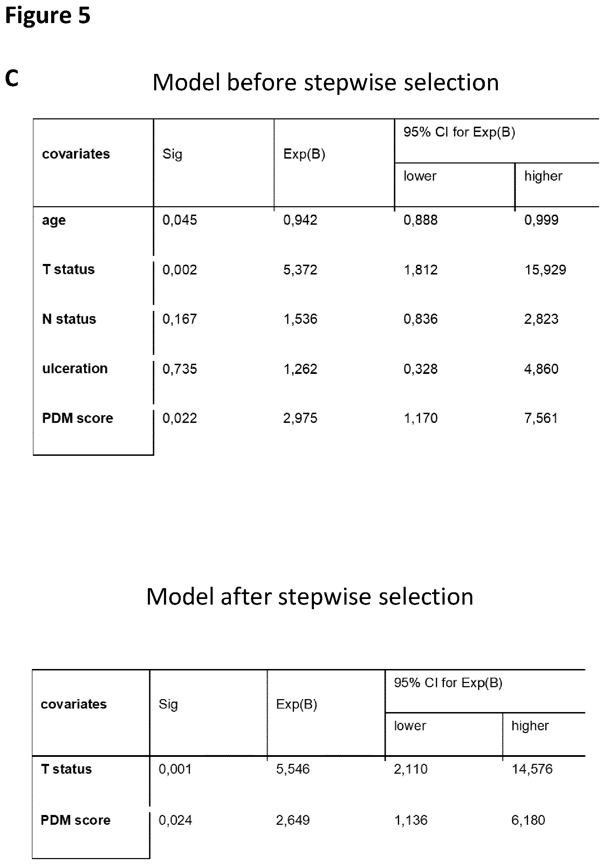

**Figure 5**. Clinical relevance of the PDM score. **A**) Kaplan Meier curves for the disease free survival of the melanoma patients with a low (PDM_{low}), intermediate (PDMᵢₙₜ) and high (PDM_{high}) PDM score. **B**) Univariate analysis of the 3 PDM score related groups along with the significance and hazard ratio. C) Multivariate cox regression analysis table, taking into account the major clinicopathological characteristics of the patients as well as the PDM score, before and after stepwise selection for the independent variables. Statistical significance and the hazard ratio (Exp(B)) with its 95 % CI are presented.

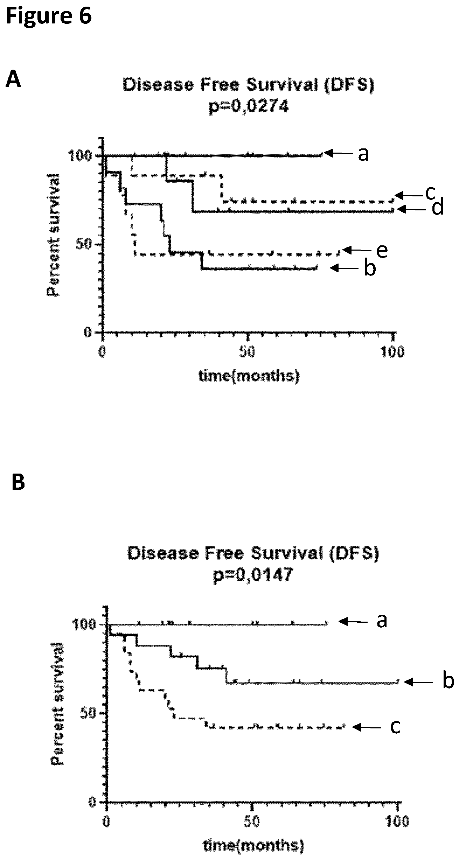

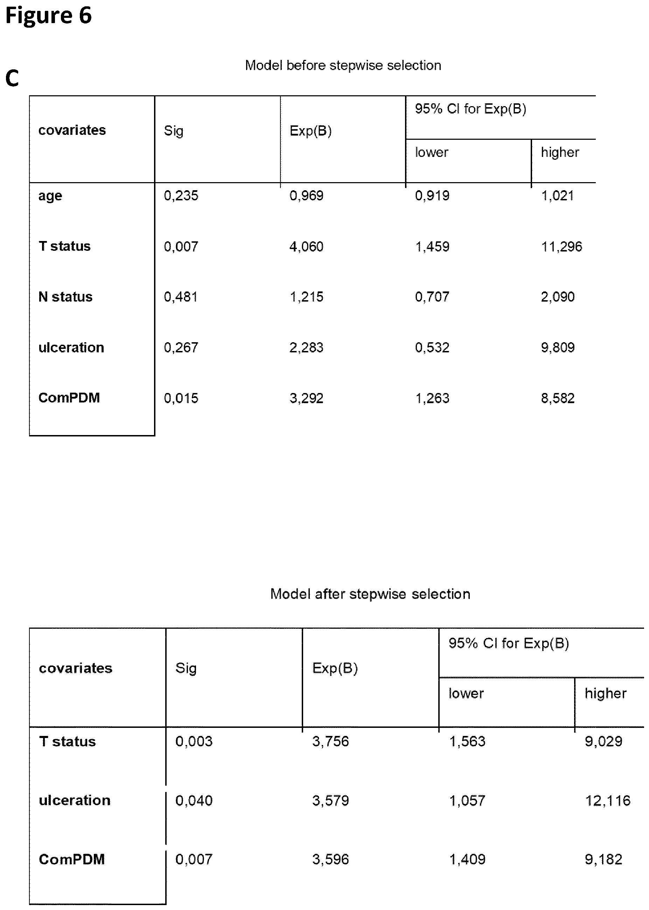

**Figure 6****.** Correlation of patients' survival by combination of the PDM score and PD-L1 expression status. **A**) Kaplan Meier curves for patients' DFS in relation to their PDM score (low in green, intermediate in blue, high in red) and PD-L1 expression status (PD-L1-negative (PD-L1⁻) with continuous lines, PD-L1-positive (PD-L1+) with dotted lines; a: PDM_{low}, b: PDMᵢₙₜ/PD-L1⁻, c: PDMᵢₙₜ/PD-L1⁺, d: PDM_{high}/PD-L1⁻, e: PDM_{high}/PD-L1⁺. **B**) Kaplan Meier curves of the patients according to their comPDM status. a: comPDM₁ group(PDM_{low} patients regardless of PD-L1 status and CD8+ T cell infiltration), b: comPDM₂ group (PDM-_{high}/PD-L1⁻ patients and PDMᵢₙₜ/CD8+high patients), c: comPDM₃ group (PDtv)_{high}/PD-L1⁺ and PDMᵢₙₜCD8+low patients. **C**) Multivariate cox regression analysis table, combining the major clinicopathological characteristics of the patients with the new comPDM score, before and after stepwise selection for the independent variables. Statistical significance and the hazard ratio (Exp(B)) with its 95%Cl are presented.

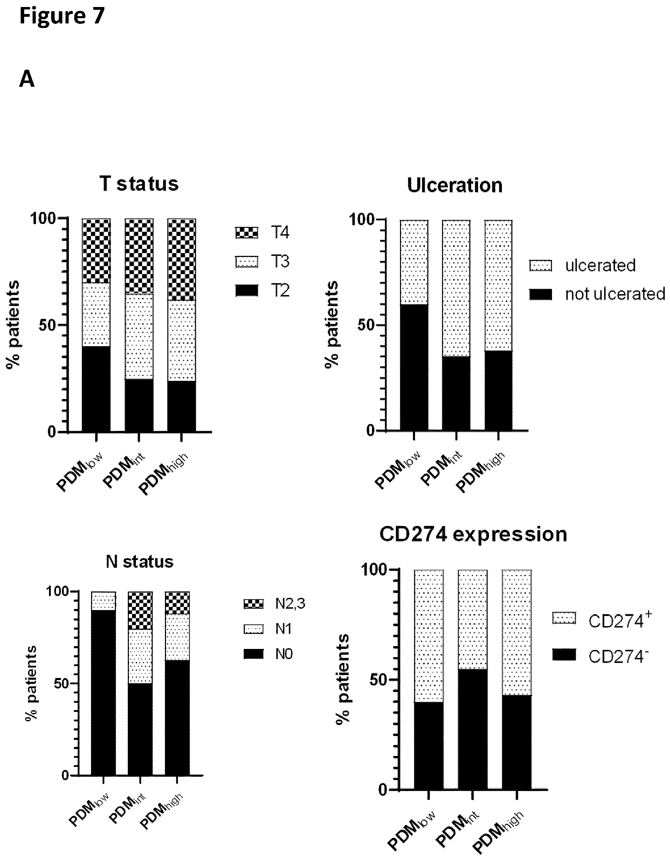

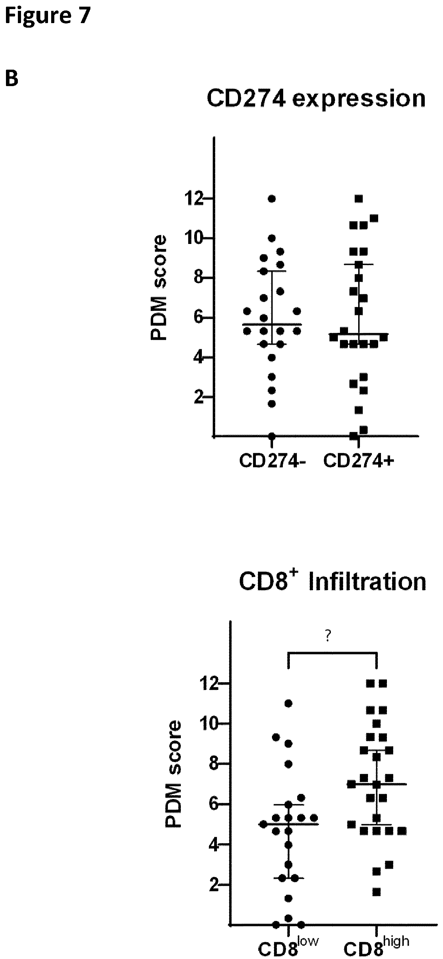

**Figure 7**. Clinicopathological characteristics of patients in relation to their PDM scores. **A**) Distribution of the clinicopathological characteristics of the patients within the 3 PDM score groups generated. **B**) Correlation of individual PDM scores of patients in the context to the PD-L1 (CD274) expression and CD8+ cell infiltration of the tumor.

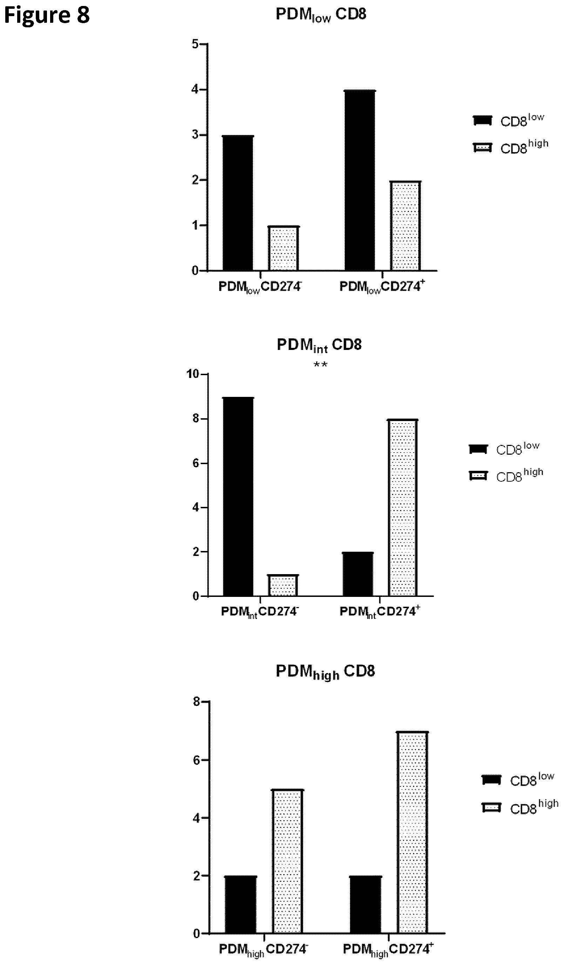

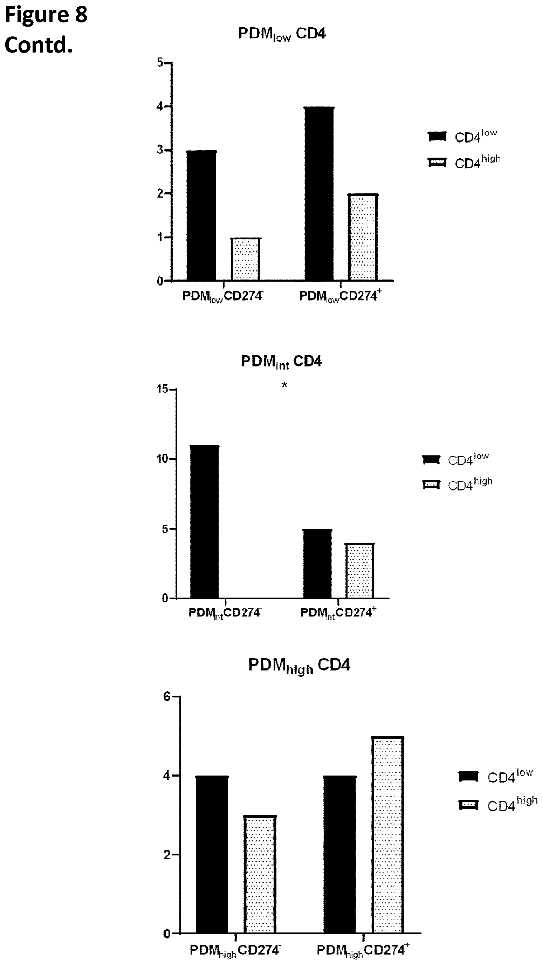

**Figure 8**. CD8+ and CD4+ T cell distribution in melanoma patients with different PDM scores and PD-L1 expression. Significant results with p<0,05 are depicted with one asterisk (*), while results with p<0,01 with two asterisks (**).

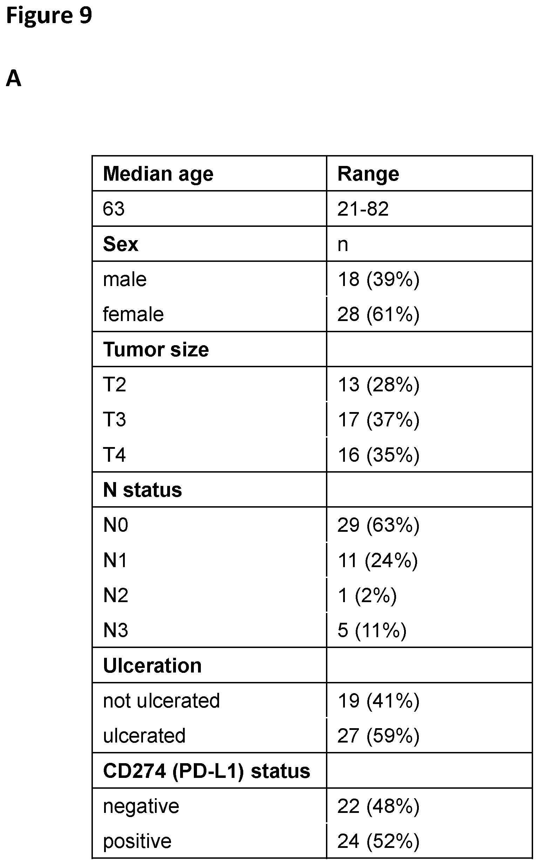

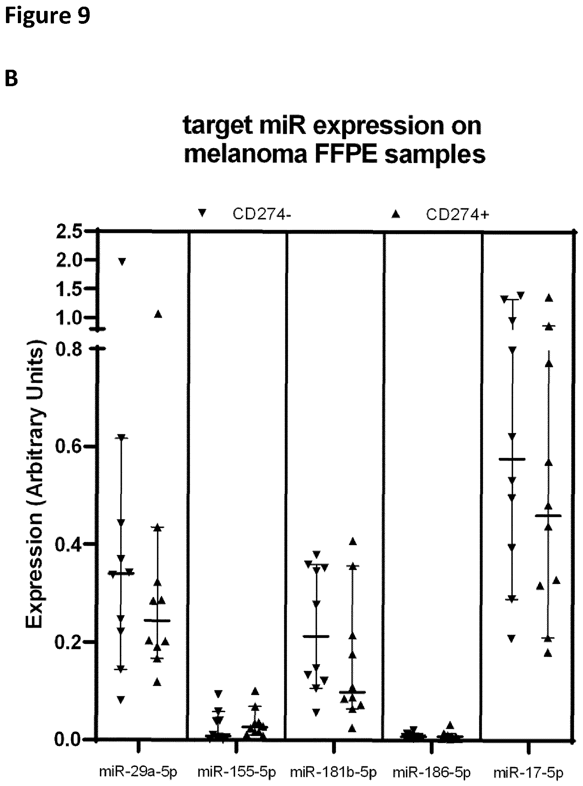

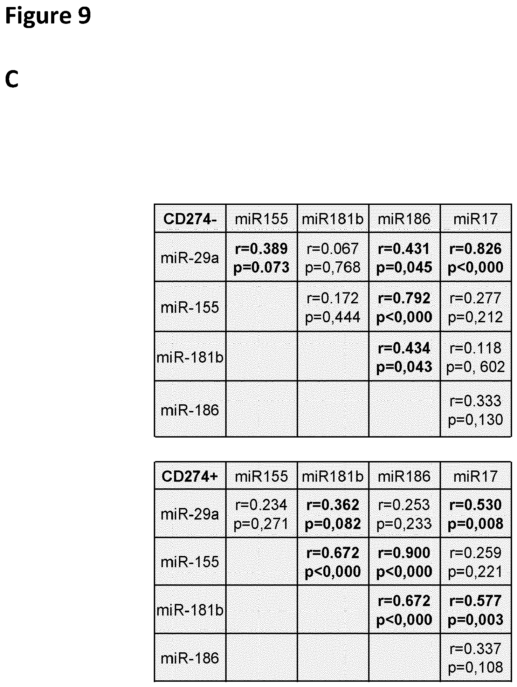

**Figure 9****.** FFPE samples from 46 melanoma patients were collected at the University Hospital of Zurich, Switzerland. **A**) The clinicopathological characteristics of the patients are summarized in the Table. **B**) Expression of the miRNA candidates in FFPE samples of melanoma upon their subdivision based on the PD-L1 expression level. Patients negative for CD274 are depicted with a down-wards facing triangle, while positive with an upward. **C**) Tables with the correlation coefficients and their statistical significance for after Pearson correlation.

### DETAILED DESCRIPTION OF THE INVENTION

### Definitions

Unless defined otherwise, all technical and scientific terms used herein have the meaning commonly understood by the person skilled in the art.

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features, in addition to the explicitly stated technical features. In the same sense, the term "involving" as well as other respective grammatical forms such as "involves" and "involved" is not limiting. The same applies for the term "including" and other grammatical forms such as "includes" and "included".

Section headings throughout the description are for organizational purposes only. In particular, they are not intended as limiting for various embodiments described therein, and it is to be understood that embodiments (and features therein) described under one subheading may be freely combined with embodiments (and features therein) described under another subheading.

Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

The term "prognosing" or other grammatical forms thereof as used herein refers to an estimate of the future development, in particular the future development of the disease. Prognosing a PD-L1-positive cancer in a patient therefore refers to estimating the future development of the PD-L1-positive cancer in the patient. Preferably, the prognosis involves determining or estimating the disease-free survival (DFS). In some embodiments, prognosing involves the determination or estimation of the progression-free survival.

The term "cancer" is used is commonly understood in the art. Preferably, the cancer is a PD-L1-positive cancer, which means that the cancer cells express PD-L1 and preferably display PD-L1 on their surface. In some embodiments, the cancer is selected from the group consisting of skin cancer, such as melanoma, non-small cell lung cancer (NSCLC), Hodg-kin's lymphoma, bladder cancer, renal cell carcinoma (RCC), head and neck squamous cell carcinoma (HNSCC), breast cancer, Merkel cell carcinoma, hepatocellular carcinoma (HCC) and gastric cancer (GC). In preferred embodiments, the cancer is a skin cancer. Preferably melanoma.

As used herein, the term "microRNA (miRNA)" refers to a small single stranded RNA nucleic acid with a length of 17-27 nucleotides, preferably 22 nucleotides, which is capable of causing gene silencing of a target gene when the miRNA is present within a cell and which comprises a sequence that is complementary to a portion of the messenger RNA (mRNA) of the target gene. The miRNA may have a length of 17, 18, 19, 20, 21, 22, 23, 24, 25, 26, or 27 nucleotides. Preferred are miRNAs with a length of 20, 21, 22, 23 or 24 nucleotides. Particularly preferred are miRNAs with a length of 22 nucleotides. The miRNA may be a pri-miRNA, a pre-miRNA a mature miRNA or any other intermediate structure commonly known to the skilled person.

As used herein, the term "miRNA mimic" refers to a modified, small, partially or completely double-stranded nucleic acid, which has the same function as a corresponding miRNA, i.e. which is capable of causing gene silencing of the target gene of the corresponding miRNA when the miRNA mimic is present within a cell and which comprises a sequence that is complementary to a portion of the messenger RNA (mRNA) of the target gene. In a miRNA mimic, the sugar moiety, phosphate and/or the base of one or more nucleotides is chemically modified, in particular covalently modified. The two strands of the miRNA mimic are referred to as "first strand" and "second strand", wherein the first strand is complementary to a portion of the mRNA of the target gene. The miRNA mimic has a length a length of about 22 nucleotides, preferably a length of 17-27 nucleotides. The miRNA mimic may have a length of 17, 18, 19, 20, 21, 22, 23, 24, 25, 26, or 27 nucleotides. Preferred are miRNA mimics with a length of 20, 21, 22, 23 or 24 nucleotides. Particularly preferred are miRNA mimics with a length of 22 nucleotides. A miRNA mimic may be defined by the sequence of nucleotides and modified nucleotides comprised in the miRNA mimic. The miRNA mimic may also consist of a sequence of first strand nucleotides and modified nucleotides as disclosed herein in combination with a corresponding second strand.

As used herein, the term "modified" and the corresponding noun "modification" refers to chemical modifications, in particular covalent modifications, of the sugar moiety, phosphate and/or the base of a nucleotide of the nucleic acid, miRNA or miRNA mimic. In particular, the 2'-hydroxyl of the ribose can be modified to protect the nucleic acid from degradation. Different chemical modifications have been developed such as phosphodiester linkages, ribose backbone, 2'-O-(2-Methoxyethyl), 2'-O-Methyl, 2'- locked nucleic acid, and 2'- Fluoro.

As used herein, the term "subject" or "patient", which are used interchangeably, refers to a mammal, without limitation, including humans and other primates (e.g., chimpanzees, cyno-mologous monkeys, and other apes and monkey species), farm animals (e.g., cattle, sheep, pigs, goats and horses), domestic mammals (e.g., dogs and cats), laboratory animals (e.g., rabbits, rodents such as mice, rats, and guinea pigs), and birds (e.g., domestic, wild and game birds such as chickens, turkeys and other gallinaceous birds, ducks, geese, and the like). In preferred embodiments, the subject is a human.

"Gene silencing" as used herein refers to the regulation of gene expression that leads to a reduction of the number of gene transcripts (mRNA) and/or a reduction of the number of translated gene products in a cell.

The term "miRNA worth", which is synonymously used with "miRNA maximum point worth", also generally referred to as a score, refers to a numerical value, e.g. 1, 2, 3, 4, 5, 6, 7, 8, 9, or 10 that corresponds to the degree of reduction that the miRNA or a miRNA mimic thereof causes in PD-L1 surface expression upon administration of the miRNA or a miRNA mimic thereof (e.g a miRNA or miRNA mimic selected from SEQ ID NO: 1-60) in cell culture to a PD-L1-positive cancer cell line, wherein the greater the reduction in PD-L1 surface expression upon administration of the miRNA or a miRNA mimic thereof, the greater the miRNA worth, wherein the miRNA worth is a positive number. The degree of reduction in PD-L 1 surface expression in cell culture upon administration of a miRNA or a corresponding miRNA mimic can be assessed as indicated in the enclosed examples. The exemplified method is not limited to the cell line used in the examples or the miRNAs used in the examples, but can be extended to other cell lines and other miRNAs. In one embodiment, the miRNA worth for miR-29 is 1*a, the miRNA worth for miR-155 is 3*a, the miRNA worth for miR-181b is 2*a, the miRNA worth for miR-186 is 3*a and the miRNA worth for miR-17 is 3*a, wherein a is a factor and a positive number, preferably 1.

The term "expression multiplier" refers to a numerical value, e.g. 0, 1/3, 2/3, or 1 that can be attributed to a miRNA, e.g. each selected miRNA, wherein the expression multiplier corresponds to the tumor expression level of the miRNA as compared to the tumor expression level of the same miRNA in a reference group of patients suffering from the same cancer, wherein the greater the expression of the miRNA in the patient relative to the reference group of patients, the greater the expression multiplier, wherein the expression multiplier is a positive number or zero.

The term "individual miRNA score", also generally referred to as a score, refers to a numerical value that is attributed to a single miRNA, e.g. a miRNA selected from the group consisting of SEQ ID NO: 1-60. The individual miRNA score of a miRNA is the product of the miRNA worth for the miRNA and the expression multiplier for the same miRNA (the result of the multiplication of the miRNA worth with the expression multiplier, miRNA worth*expression multiplier). For instance, if the miRNA worth is 3 and the expression multiplier is 1/3 then the individual miRNA score is 1.

The term "PDM score" is the sum of a number of individual miRNA scores, for instance 2, 3, 4, 5, 6, 7, 8, 9, 10, 11, 12, 13, 14, 15 or more individual miRNA scores. For instance, the PDM score can be the sum of 2, 3, 4, 5, 6, 7, 8, 9, 10, 11, 12, 13, 14, 15 or more individual miRNA scores of the miRNAs according to SEQ ID NO: 1-60. The sum of a number of individual miRNA scores is formed by addition of said scores. The PDM score is a numerical value. The value depends on the number of individual miRNA scores that are used for the PDM score and the way the individual miRNA scores are calculated. Preferably, the PDM score ranges from 0 to 12, including 0 and 12. The PDM score is a prognostic factor that allows to make an estimation about the future development of a PD-L1-positive cancer in a patient. In particular, the PDM score is correlated with disease-free survival. Preferably, an intermediate to high PDM score is associated with a significantly lower disease-free survival of a PD-L 1-postive cancer patient. A low PDM score (PDM_{low}) can be a PDM score ranging from 0 to 3 (0< PDM_{low}≤3), a intermediate PDM score (PDMᵢₙₜ) can be a PDM score ranging from 3 (not including 3) to 7 (3<PDMᵢₙₜ≤7) and a high PDM score (PDM_{high}) can be a PDM score ranging from 7 (not including 7) to 12 (7<PDM_{high}≤12). In a preferred embodiment, the PDM score for the group of miRNAs miR-29, miR-155, miR-181b, miR-186 and miR-17 as determined for a patient suffering from a PD-L1-positive cancer ranges from 0 to 12, including 0 and 12.

In one aspect the miRNAs, in particular the miRNAs selected from the group consisting of SEQ ID NO: 1-60 are used as biomarkers. Preferably, the miRNAs are used as biomarkers for prognosing PD-L1 positive cancer. In one embodiment, a group of miRNAs selected from the group consisting of SEQ ID NO: 1-60 is used as biomarkers as indicated herein. In one embodiment at least one miRNA selected from the group consisting of SEQ ID NO: 1-60 is used as a biomarker for prognosing PD-L1-positive cancer in a patient.

Further provided is a kit for prognosing PD-L1 positive cancer, or in the kit comprises components to detect the expression level of one or more of the miRNAs of SEQ ID NO: 1-60. Preferably, the component is a polymerase chain reaction primer, or primer pair, that allows the detection of the miRNA in a polymerase chain reaction (PCR). Alternatively, the kit may comprise a MicroArray for detecting the expression of one or more of the miRNAs of SEQ ID NO: 1-60. Typically, the kit contains an instruction leaflet for carrying out the method of the invention.

In some embodiments, the method of prognosing PD-L1-positive cancer in a patient can be used to assess or monitor immunotherapeutic treatment of the patient. For instance, the method can be used to assess or monitor treatment with a checkpoint inhibitor, such as a PD-1 or PD-L1 inhibitor. In particular, the method can be used to assess or monitor treatment with any one of Nivolumab, Pembrolizumab, Atezolizumab, Avelumab, Durvalumab, Cemiplimab, and Dostarlimab. Similarly, the uses and kits taught herein can be used for assessing and monitoring immunotherapeutic treatment as indicated herein.

The methods, uses and kits herein are in particular based on and make use of the following miRNAs as indicated in Table 1:

**Table 1: miRNA sequences**

| **miRNA name** | **Sequence of first strand** | **SEQ ID NOs** |
|---|---|---|
| hsa-mir-26a-5p | UUCAAGUAAUCCAGGAUAGGCU | SEQ ID NO: 1 |
| hsa-miR-26b-5p | UUCAAGUAAUUCAGGAUAGGU | SEQ ID NO: 2 |
| hsa-miR-29a-3p | UAGCACCAUCUGAAAUCGGUUA | SEQ ID NO: 3 |
| hsa-miR-103b | UCAUAGCCCUGUACAAUGCUGCU | SEQ ID NO: 4 |
| hsa-miR-181b-5p | AACAUUCAUUGCUGUCGGUGGGU | SEQ ID NO: 5 |
| hsa-miR-186-5p | CAAAGAAUUCUCCUUUUGGGCU | SEQ ID NO: 6 |
| hsa-miR-155-5p | UUAAUGCUAAUCGUGAUAGGGGUU | SEQ ID NO: 7 |
| hsa-miR-17-5p | CAAAGUGCUUACAGUGCAGGUAG | SEQ ID NO: 8 |
| hsa-miR-140-5p | CAGUGGUUUUACCCUAUGGUAG | SEQ ID NO: 9 |
| hsa-miR-425-5p | AAUGACACGAUCACUCCCGUUGA | SEQ ID NO: 10 |
| hsa-miR-30b-5p | UGUAAACAUCCUACACUCAGCU | SEQ ID NO: 11 |
| hsa-miR-574-3p | CACGCUCAUGCACACACCCACA | SEQ ID NO: 12 |
| hsa-miR-29b-3p | UAGCACCAUUUGAAAUCAGUGUU | SEQ ID NO: 13 |
| hsa-miR-9-5p | UCUUUGGUUAUCUAGCUGUAUGA | SEQ ID NO: 14 |
| hsa-miR-320b | AAAAGCUGGGU UGAGAGGGCAA | SEQ ID NO: 15 |
| hsa-miR-25-3p | CAUUGCACUUGUCUCGGUCUGA | SEQ ID NO: 16 |
| hsa-miR-103a-3p | AGCAGCAUUGUACAGGGCUAUGA | SEQ ID NO: 17 |
| hsa-miR-320c | AAAAGCUGGGUUGAGAGGGU | SEQ ID NO: 18 |
| hsa-miR-629-5p | UGGGUUUACGUUGGGAGAACU | SEQ ID NO: 19 |
| hsa-miR-24-3p | UGGCUCAGUUCAGCAGGAACAG | SEQ ID NO: 20 |
| hsa-miR-3074-5p | GUUCCUGCUGAACUGAGCCAG | SEQ ID NO: 21 |
| hsa-miR-532-3p | CCUCCCACACCCAAGGCUUGCA | SEQ ID NO: 22 |
| hsa-miR-181a-5p | AACAUUCAACGCUGUCGGUGAGU | SEQ ID NO: 23 |
| hsa-miR-27b-3p | UUCACAGUGGCUAAGUUCUGC | SEQ ID NO: 24 |
| hsa-miR-30c-5p | UGUAAACAUCCUACACUCUCAGC | SEQ ID NO: 25 |
| hsa-miR-151a-3p | CUAGACUGAAGCUCCUUGAGG | SEQ ID NO: 26 |
| hsa-miR-4521 | GCUAAGGAAGUCCUGUGCUCAG | SEQ ID NO: 27 |
| hsa-miR-106b-3p | CCGCACUGUGGGUACUUGCUGC | SEQ ID NO: 28 |
| hsa-miR-23a-3p | AUCACAUUGCCAGGGAUUUCC | SEQ ID NO: 29 |
| hsa-miR-20a-5p | UAAAGUGCUUAUAGUGCAGGUAG | SEQ ID NO: 30 |
| hsa-miR-146b-5p | UGAGAACUGAAUUCCAUAGGCUG | SEQ ID NO: 31 |
| hsa-miR-3074-5p | GUUCCUGCUGAACUGAGCCAG | SEQ ID NO: 32 |
| hsa-miR-30e-5p | UGUAAACAUCCUUGACUGGAAG | SEQ ID NO: 33 |
| hsa-miR-222-3p | AGCUACAUCUGGCUACUGGGU | SEQ ID NO: 34 |
| hsa-miR-340-5p | UUAUAAAGCAAUGAGACUGAUU | SEQ ID NO: 35 |
| hsa-miR-584-5p | UUAUGGUUUGCCUGGGACUGAG | SEQ ID NO: 36 |
| hsa-miR-550a-3-5p | AGUGCCUGAGGGAGUAAGAG | SEQ ID NO: 37 |
| hsa-miR-550a-5p | AGUGCCUGAGGGAGUAAGAGCCC | SEQ ID NO: 38 |
| hsa-miR-454-3p | UAGUGCAAUAUUGCUUAUAGGGU | SEQ ID NO: 39 |
| hsa-let-7g-5p | UGAGGUAGUAGUUUGUACAGUU | SEQ ID NO: 40 |
| hsa-miR-122-5p | UGGAGUGUGACAAUGGUGUUUG | SEQ ID NO: 41 |
| hsa-miR-3591-3p | AAACACCAUUGUCACACUCCAC | SEQ ID NO: 42 |
| hsa-miR-27a-3p | UUCACAGUGGCUAAGUUCCGC | SEQ ID NO: 43 |
| hsa-miR-185-3p | AGGGGCUGGCUUUCCUCUGGUC | SEQ ID NO: 44 |
| hsa-miR-25-5p | AGGCGGAGACUUGGGCAAUUG | SEQ ID NO: 45 |
| hsa-let-7e-5p | UGAGGUAGGAGGUUGUAUAGUU | SEQ ID NO: 46 |
| hsa-miR-211-5p | UUCCCUUUGUCAUCCUUCGCCU | SEQ ID NO: 47 |
| hsa-miR-221-3p | AGCUACAUUGUCUGCUGGGUUUC | SEQ ID NO: 48 |
| hsa-let-7f-5p | UGAGGUAGUAGAUUGUAUAGUU | SEQ ID NO: 49 |
| hsa-miR-128-3p | UCACAGUGAACCGGUCUCUUU | SEQ ID NO: 50 |
| hsa-miR-93-5p | CAAAGUGCUGUUCGUGCAGGUAG | SEQ ID NO: 51 |
| hsa-let-7i-5p | UGAGGUAGUAGUUUGUGCUGUU | SEQ ID NO: 52 |
| hsa-let-7c-5p | UGAGGUAGUAGGUUGUAUGGUU | SEQ ID NO: 53 |
| hsa-miR-378a-3p | ACUGGACUUGGAGUCAGAAGGC | SEQ ID NO: 54 |
| hsa-miR-105-5p | UCAAAUGCUCAGACUCCUGUGGU | SEQ ID NO: 55 |
| hsa-let-7b-5p | UGAGGUAGUAGGUUGUGUGGUU | SEQ ID NO: 56 |
| hsa-miR-193a-5p | UGGGUCUUUGCGGGCGAGAUGA | SEQ ID NO: 57 |
| hsa-miR-451a | AAACCGUUACCAUUACUGAGUU | SEQ ID NO: 58 |
| hsa-miR-103a-2-5p | AGCUUCUUUACAGUGCUGCCUUG | SEQ ID NO: 59 |
| hsa-miR-550b-3p | UCUUACUCCCUCAGGCACUG | SEQ ID NO: 60 |

These miRNAs have been identified as PD-L1 regulating miRNAs following the approaches as described in the examples.

In an embodiment, the miRNA is selected from any one of SEQ ID NO: 1-16. In a preferred embodiment, the miRNA is one or more of miR-29, miR-155, miR-181b, miR-186 and miR-17. Preferably, all five miRNAs of miR-29, miR-155, miR-181b, miR-186 and miR-17, e.g. their tumor expression level is measured in a sample from the patient or they are used as biomarkers or PCR primers capable of detecting all five miRNAs miR-29, miR-155, miR-181b, miR-186 and miR-17 are included in the kit or a MicroArray capable of detecting all five miRNAs miR-29, miR-155, miR-181b, miR-186 and miR-17 is included in the kit.

In a preferred embodiment, the miRNA is capable of reducing gene expression of PD-L1 by gene silencing, preferably, wherein the gene silencing by the miRNA reduces translation of PD-L1.

In an embodiment, the miRNA targets one, two or three regions, preferably at least two regions, of the PD-L1 transcript selected from the coding region (CDS), the 3' UTR-1 and the 3'UTR-2 of PD-L1 as defined in SEQ ID NOs: 62-64.

In a preferred embodiment, the miRNA is capable of hybridizing to both the 3' UTR and the CDS of a PD-L1 mRNA.

In an embodiment, "targeting" or "targets" or other grammatical forms thereof means capable of hybridizing to the CDS, the 3' UTR-1 and/or the 3'UTR-2 of PD-L1.

In an embodiment, the miRNA of the invention targets the CDS, the 3' UTR-1 and the 3'UTR-2 of PD-L1 as defined in SEQ ID NOs: 62-64.

In an embodiment the miRNA is capable of hybridizing to the 3' UTR (SEQ ID NO: 63 or 64) and is selected from hsa-miR-103a-3p, hsa-miR-106b-3p, hsa-miR-140-5p, hsa-miR-146b-5p, hsa-miR-151a-3p, hsa-miR-181a-5p, hsa-miR-20a-5p, hsa-miR-222-3p, hsa-miR-23a-3p, hsa-miR-24-3p, hsa-miR-25-3p, hsa-miR-27b-3p, hsa-miR-29b-3p, hsa-miR-3074-5p, hsa-miR-30b-5p, hsa-miR-30c-5p, hsa-miR-30e-5p, hsa-miR-320b, hsa-miR-320c, hsa-miR-340-5p, hsa-miR-425-5p, hsa-miR-4521, hsa-miR-532-3p, hsa-miR-574-3p, hsa-miR-629-5p, and hsa-miR-9-5p.

In an embodiment the miRNA is capable of hybridizing to the 3' UTR-1 of PD-L1 (SEQ ID NO: 63) and is selected from the group consisting of hsa-miR-140-5p, hsa-miR-425-5p, hsa-miR-30b-5p, hsa-miR-574-3p, hsa-miR-29b-3p, hsa-miR-9-5p, hsa-miR-320b, hsa-miR-25-3p, hsa-miR-103a-3p, hsa-miR-320c, hsa-miR-629-5p, hsa-miR-24-3p, hsa-miR-3074-5p, hsa-miR-532-3p, hsa-miR-181a-5p, hsa-miR-27b-3p, hsa-miR-30c-5p, hsa-miR-151a-3p, hsa-miR-4521, hsa-miR-106b-3p and hsa-miR-23a-3p.

In an embodiment the miRNA is capable of hybridizing to the 3' UTR-2 of PD-L1 (SEQ ID NO: 64) and is selected from the group consisting of hsa-miR-20a-5p, hsa-miR-146b-5p, hsa-miR-3074-5p, hsa-miR-24-3p, hsa-miR-151a-3p, hsa-miR-30e-5p, hsa-miR-103a-3p, hsa-miR-222-3p and hsa-miR-340-5p.

In an embodiment the miRNA is capable of hybridizing to the CDS of PD-L1 (SEQ ID NO: 62) and is selected from the group consisting of hsa-miR-584-5p, hsa-miR-9-5p, hsa-miR-550a-3-5p, hsa-miR-550a-5p, hsa-miR-454-3p, hsa-miR-27b-3p, hsa-let-7g-5p, hsa-miR-181a-5p, hsa-miR-122-5p, hsa-miR-3591-3p, hsa-miR-20a-5p, hsa-miR-27a-3p, hsa-miR-103a-3p, hsa-miR-185-3p, hsa-miR-25-5p, hsa-miR-222-3p, , hsa-miR-629-5p, hsa-miR-4521, hsa-let-7e-5p, hsa-miR-211-5p, hsa-miR-221-3p, hsa-let-7f-5p, hsa-miR-128-3p, hsa-miR-93-5p, hsa-let-7i-5p, hsa-let-7c-5p, hsa-miR-23a-3p, hsa-miR-378a-3p, hsa-miR-105-5p, hsa-let-7b-5p, hsa-miR-193a-5p, hsa-miR-451a, hsa-miR-103a-2-5p and hsa-miR-550b-3p.

In an embodiment, the miRNA is capable of targeting PD-L1, wherein the miRNA or a corresponding miRNA mimic comprises a conserved region at the 5' end of the miRNA or miRNA mimic, in nucleotides 2-7, 3-8, 3-7, 1-7, or 1-8, as counted from the 5' end of the miRNA or corresponding miRNA mimic, and wherein the conserved region has a perfect match with the target sequence.

In a preferred embodiment, the miRNA targeting the CDS, the 3' UTR-1 and the 3'UTR-2 of PD-L1 is selected from any one of hsa-miR-103a-3p, hsa-miR-103b, hsa-miR-107, hsa-miR-148a-3p, hsa-miR-148b-3p, hsa-miR-152-3p, hsa-miR-181a-5p, hsa-miR-181b-5p, hsa-miR-211-5p, hsa-miR-23a-3p, hsa-miR-25-5p, hsa-miR-34a-5p, hsa-miR-454-3p, hsa-miR-584-5p and hsa-miR-629-5p as shown in Table 1 above. In a more preferred embodiment, the miRNA is selected from any one of hsa-miR-103a-3p, hsa-miR-103b, hsa-miR-148a-3p, hsa-miR-148b-3p, hsa-miR-152-3p, hsa-miR-181a-5p, hsa-miR-181b-5p and hsa-miR-34a-5p.

In an embodiment, the miRNA of the invention targets both the CDS and the 3'UTR-2 of PD-L1 as defined in SEQ ID Nos: 62 and 64. In a preferred embodiment, the miRNA targeting both the CDS and the 3'UTR-2 of PD-L1 is selected from hsa-miR-105-5p, hsa-miR-17-5p, hsa-miR-20a-5p, hsa-miR-222-3p, hsa-miR-451a, hsa-miR-486-3p, hsa-miR-486-5p, and hsa-miR-93-5p as shown in Table 1 above.

In a further embodiment, the miRNA of the invention targets both the CDS and the 3'UTR-1 of PD-L1 as shown in Table 2 (SEQ ID NOs: 62 and 63). In an embodiment, targeting means capable of hybridizing to the 3'UTR of PD-L1. In an embodiment, the miRNA targets the second half (i.e. the downstream half or the 3' half) of the 3'UTR of the mRNA of PD-L1. In a preferred embodiment, the miRNA targets the 3'UTR-2 sequence as shown in Table 2 (SEQ ID NO: 64).

In an embodiment, the miRNA of the invention targets both the 3'UTR-1 and the 3'UTR-2 of PD-L1 as defined in SEQ ID Nos: 63 and 64. In a preferred embodiment, the miRNA targeting both the 3'UTR-1 and the 3'UTR-2 of PD-L1 is selected from hsa-miR-135b-5p, hsa-miR-140-5p, hsa-miR-151a-3p, hsa-miR-24-3p, hsa-miR-26b-5p, hsa-miR-27b-5p, hsa-miR-3074-5p, hsa-miR-340-5p and hsa-miR-509-3p as shown in Table 1 above. In a preferred embodiment, the miRNA as defined above is selected from hsa-miR-26b-5p and hsa-miR-340-5p.

In an embodiment, the miRNA of the invention targets the coding region (CDS) of PD-L1. In an embodiment, "targeting" means capable of hybridizing to the CDS of PD-L1. In a preferred embodiment, the miRNA targets the CDS sequence as shown in Table 2 (SEQ ID NO: 62).

In a further embodiment, the miRNA of the invention targets both the CDS and the 3'UTR of PD-L1. In an embodiment, targeting means capable of hybridizing to both the CDS and the 3'UTR of PD-L1. In an embodiment, the miRNA of the invention targets the 3'UTR of PD-L1, preferably the miRNA targets both the 3'UTR-1 and the 3'UTR-2. In a more preferred embodiment, the miRNA of the invention targets the CDS of PD-L1.

In a preferred embodiment, the miRNA targeting both the CDS and 3'UTR-1 comprises the sequence of miR-103 and/or the sequence of miR-186-5p as shown in Table 1.

In an embodiment, the miRNA is capable of reducing gene expression of PD-L1. In an embodiment, the miRNA is capable of regulating gene expression in a variety of manners, including translational repression, mRNA cleavage, and/or deadenylation. In a preferred embodiment, the miRNA is capable of inducing PD-L1 downregulation, reducing gene expression of PD-L1 by gene silencing. Preferably, the nucleic acid is capable of reducing gene translation via mRNA cleavage, deadenylation and/or RNA methylation.

In an embodiment, the miRNA of the invention is capable of co-regulating PD-L1 expression and other genes of the PD-1/PD-L1 pathway, such as c-FOS, in PD-L1⁺ cancers.

**Table 2. Full PD-L1 RNA sequence (transcript variant 1)**

| >NM_014143.4 Homo sapiens CD274 molecule (CD274), transcript variant 1, mRNA | | |
|---|---|---|
| **Region** | **Sequence** | **SEQ ID NO** |
| 5'UTR | agttctgcgcagcttcccgaggctccgcac- cagccgcgcttctgtccgcctgcaggg- cattccagaaag | SEQ ID NO: 61 |
| CDS | atgaggatatttgctgtctttatattcatgac- ctactggcatttgctgaac- gcatttactgtcacggttcccaaggacctatatgtgg tagagtatggtagcaatatgacaatt- gaatgcaaattcccagtagaaaaacaattagac- ctggctgcactaattgtctattgggaaatggaggata agaacattattcaatttgtgcatggagaggaagac- ctgaaggttcagcatagtagctacaga- cagagggcccggctgttgaaggaccagctctccctgg gaaatgctgcacttcagatcacagatgtgaaatt- gcaggatgcaggggtgtaccgctg- catgatcagctatggtggtgccgactacaagcgaatt actgtgaaagtcaatgccccatacaacaaaatcaac- caaagaattttggttgtggatccag- tcacctctgaacatgaactgacatgtcaggctgaggg ctaccccaaggccgaagtcatctggacaagcag- tgaccatcaagtcctgagtggtaagac- caccaccaccaattccaagagagaggagaagcttttc aatgtgaccagcacactga- gaatcaacacaacaactaatgagattttctactg- cacttttag- gagattagatcctgaggaaaaccatacagctgaattg gtcatcccagaactacctctgg- cacatcctccaaatgaaaggactcacttgg- taattctgg- gagccatcttattatgccttggtgtagcactgacatt catcttccgtttaagaaaaggga- gaatgatggatgtgaaaaaatgtggcatccaagata- caaactcaaagaagcaaagtgatacacatttggagga gacgtaa | SEQ ID NO: 62 |
| 3'UTR-1 | tccagcattggaacttctgatcttcaagcagggat- tctcaac- ctgtggtttaggggttcatcggggctgagcgtgacaagag gaaggaatgggcccgtgggatgcaggcaatgtgg- gacttaaaaggcccaagcac- tgaaaatggaacctggcgaaagcagaggaggagaatgaag aaagatggagtcaaacagggagcctggagggagacctt- | SEQ ID NO: 63 |
| | ga- tactttcaaatgcctgaggggctcatcgacgcctgtgaca gggagaaaggatacttctgaacaaggagcctccaa- gcaaatcatccattgctcatcctag- gaagacgggttgagaatccctaatttgagggtcagttcct gcagaagtgccctttgcctccactcaatcctcaattt- gttttctgcatgactgagag- tctcagtgttggaacgggacagtatttatgtatgagtttt tcctatttattttgagtctgtgaggtcttctt- gtcatgtgagtgtggttgtgaatgat- ttcttttgaagatatattgtagtagatgttacaattttgt cgccaaactaaacttgctgcttaatgattt- gctcacatctagtaaaacatggagtattt- gtaaggtgcttggtctcctctataactacaagtatacatt ggaagcataaagatcaaaccgttggtt- gcataggatgtcacctttatttaacccattaa- tactctggttgacctaatcttattctcagacctcaagtgt ctgtgcagtatctgttccatttaaa- tatcagctttacaattatgtggtagcc- tacacacataatctcatttcatcgctgtaaccaccctgtt gtgataaccactattattttacccatcgtacagctgag- gaagcaaacagattaagtaactt- gcccaaaccagtaaatagcagacctcagactgccacccac tgtccttttataatacaatttacagcta- tattttactttaagcaattcttttattcaaaaac- catttattaagtgcccttgcaatatcaatcgctgtgccag gcattgaatctacagatgtgagcaagacaaagtac- ctgtcctcaaggagctcatagtataatgaggagattaaca agaaaatgtattattacaatttagtccag- tgtcatagcataaggatgatgcgaggg- gaaaacccgagcagtgttgccaagaggaggaaataggcca atgtggtctgggacggttggatatacttaaacatcttaa- taatcagag- taattttcatttacaaagagaggtcggtacttaaaataac cctgaaaaataacactggaattccttttctagcatta- tatttattcctgatttgcctttt- gccatataatctaatgcttgtttatatagtgtctggtatt gtttaacagttctgtct | |
| 3' UTR-2 | ggtacttaaaataaccctgaaaaataacactg- gaattccttttctagcatta- tatttattcctgatttgcctttgccatataatctaatgct tgtttatatagtgtctggtattgtttaacag- ttctgtcttttctatttaaatgccac- taaattttaaattcatacctttccatgattcaaaattcaa aagatcccatgggagatggttggaaaatctccac- ttcatcctccaa- gccattcaagtttcctttccagaagcaactgctactgcct ttcattcatatgttcttctaaagatagtctacattt- ggaaatgtatgttaaaagcac- gtatttttaaaatttttttcctaaatagtaacacattgta tgtctgctgtgtactttgctatttttatttattttag- tgtttcttata- tagcagatggaatgaatttgaagttcccagggctgaggat ccatgccttctttgtttctaagttatctttccca- | SEQ ID NO: 64 |
| | tagcttttcattatctttcat- atgatccagtatatgttaaatatgtcctacatatacattt agacaaccaccatttgttaagtatttgctctag- gacagagtttggatttgtttatgttt- gctcaaaaggagacccatgggctctccagggtgcactgag tcaatctagtcctaaaaagcaatcttattattaactctg- tatgacagaatcatgtctg- gaacttttgttttctgctttctgtcaagtataaacttcac tttgatgctgtacttgcaaaatcacattttctttctg- gaaattccggcagtgtacctt- gactgctagctaccctgtgccagaaaagcctcattcgttg tgcttgaacccttgaatgccaccagctgtcatcac- tacacagccctcctaagaggcttcctg- gaggtttcgagattcagatgccctgggagatcccagagtt tcctttccctctt- ggccatattctggtgtcaatgacaaggagtaccttt- ggcttt- gccacatgtcaaggctgaagaaacagtgtctccaacagag ctccttgtgttatctgtttgtacatgtgcatttgtacag- taattggtgtgacagtgttcttt- gtgtgaattacaggcaagaattgtggctgagcaaggcaca tagtctactcagtctattcctaagtcctaactcctcctt- gtggtgttggatttgtaaggcac- tttatcccttttgtctcatgtttcatcgtaaatggcatag gcagagatgatacctaattctgcatttgattgtcac- tttttgtacctgcattaatttaa- taaaatattcttatttattttgttacttggtacaccagca tgtccattttcttgtttattttgtgtttaa- taaaatgttcagtttaacatccca | |
| PD-L1 mRNA Full sequence | agttctgcgcagcttcccgaggctccgcac- cagccgcgcttctgtccgcctgcaggg- cattccagaaagatgaggatatttgctgtctttatattca tgacctactggcatttgctgaacgcatttactgtcac- ggttcccaaggacctatatgtgg- tagagtatggtagcaatatgacaattgaatgcaaattccc agtagaaaaacaattagacctggctgcactaattgtc- tattgggaaatggaggataa- gaacattattcaatttgtgcatggagaggaagacctgaag gttcagcatagtagctacagacagagggcccggctgtt- gaaggaccagctctccctgg- gaaatgctgcacttcagatcacagatgtgaaattgcagga tgcaggggtgtaccgctgcatgatcagc- tatggtggtgccgactacaa- gcgaattactgtgaaagtcaatgccccatacaacaaaatc aaccaaagaattttggttgtggatccagtcac- ctctgaacatgaactgacatgtcaggctgagggctacccc aaggccgaagtcatctggacaagcagtgac- catcaagtcctgagtggtaagaccaccaccac- caattccaagagagaggagaagcttttcaatgtgaccagc acactgagaatcaacacaacaactaatgagattttc- tactgcacttttagga- gattagatcctgaggaaaaccatacagctgaattggtcat cccagaactacctctgg- cacatcctccaaatgaaaggactcacttggtaattctgg- gagccatcttattatgccttggtgtagcactgacattcat | SEQ ID NO:130 |
| | cttccgtttaagaaaaggga- gaatgatggatgtgaaaaaatgtggcatccaagata- caaactcaaagaagcaaagtgatacacatttggaggagac gtaatccagcattggaacttctgatcttcaagcagggat- tctcaac- ctgtggtttaggggttcatcggggctgagcgtgacaagag gaaggaatgggcccgtgggatgcaggcaatgtgg- gacttaaaaggcccaagcac- tgaaaatggaacctggcgaaagcagaggaggagaatgaag aaagatggagtcaaacagggagcctggagggagacctt- ga- tactttcaaatgcctgaggggctcatcgacgcctgtgaca gggagaaaggatacttctgaacaaggagcctccaa- gcaaatcatccattgctcatcctag- gaagacgggttgagaatccctaatttgagggtcagttcct gcagaagtgccctttgcctccactcaatcctcaattt- gttttctgcatgactgagag- tctcagtgttggaacgggacagtatttatgtatgagtttt tcctatttattttgagtctgtgaggtcttctt- gtcatgtgagtgtggttgtgaatgat- ttcttttgaagatatattgtagtagatgttacaattttgt cgccaaactaaacttgctgcttaatgattt- gctcacatctagtaaaacatggagtattt- gtaaggtgcttggtctcctctataactacaagtatacatt ggaagcataaagatcaaaccgttggtt- gcataggatgtcacctttatttaacccattaa- tactctggttgacctaatcttattctcagacctcaagtgt ctgtgcagtatctgttccatttaaa- tatcagctttacaattatgtggtagcc- tacacacataatctcatttcatcgctgtaaccaccctgtt gtgataaccactattattttacccatcgtacagctgag- gaagcaaacagattaagtaactt- gcccaaaccagtaaatagcagacctcagactgccacccac tgtccttttataatacaatttacagcta- tattttactttaagcaattcttttattcaaaaac- catttattaagtgcccttgcaatatcaatcgctgtgccag gcattgaatctacagatgtgagcaagacaaagtac- ctgtcctcaaggagctcatagtataatgaggagattaaca agaaaatgtattattacaatttagtccag- tgtcatagcataaggatgatgcgaggg- gaaaacccgagcagtgttgccaagaggaggaaataggcca atgtggtctgggacggttggatatacttaaacatcttaa- taatcagag- taattttcatttacaaagagaggtcggtacttaaaataac cctgaaaaataacactggaattccttttctagcatta- tatttattcctgatttgccttt- gccatataatctaatgcttgtttatatagtgtctggtatt gtttaacagttctgtcttttctatttaaatgccac- taaattttaaattcat- acctttccatgattcaaaattcaaaagatcccatgggaga tggttggaaaatctccacttcatcctccaa- gccattcaagtttcctttccagaagcaactgc- tactgcctttcattcatatgttcttctaaagatagtctac atttggaaatgtatgttaaaagcac- gtatttttaaaatttttttcctaaatagtaacacatt- gtatgtctgctgtgtactttgctatttttatttattttag tgtttcttatatagcagatggaatgaattt- gaagttcccagggctgag- gatccatgccttctttgtttctaagttatctttcccatag | |
| | cttttcattatctttcatatgatccagtatatgttaaa- tatgtcctacatatacatttaga- caaccaccatttgttaagtatttgctctaggacagagttt ggatttgtttatgtttgctcaaaagga- gacccatgggctctccagggtgcactgag- tcaatctagtcctaaaaagcaatcttattattaactctgt atgacagaatcatgtctggaactttt- gttttctgctttctgtcaagtataaacttcacttt- gatgctgtacttgcaaaatcacattttctttctggaaatt ccggcagtgtaccttgactgctagc- taccctgtgccagaaaagcctcattcgttgtgctt- gaacccttgaatgccaccagctgtcatcactacacagccc tcctaagaggcttcctggaggtttcgagat- tcagatgccctgggagatcccagag- tttcctttccctcttggccatattctggtgtcaatgacaa ggagtaccttggctttgccacatgtcaaggctgaa- gaaacagtgtctccaacagagctcctt- gtgttatctgtttgtacatgtgcatttgtacagtaattgg tgtgacagtgttctttgtgtgaattacaggcaagaatt- gtggctgagcaaggcacatagtc- tactcagtctattcctaagtcctaactcctccttgtggtg ttggatttgtaaggcactttatccctttt- gtctcatgtttcatcgtaaatggcataggcaga- gatgatacctaattctgcatttgattgtcactttttgtac ctgcattaatttaataaaatattcttatttatttt- gttacttggtacaccag- catgtccattttcttgtttattttgtgtttaataaaatgt tcagtttaacatccca | |

### EXAMPLES

The following examples are provided for purposes of illustration only and are not intended to be limiting unless otherwise specified. Thus, the invention should in no way be construed as being limited to the following examples. Rather, the examples should be construed to encompass any and all variations which become evident as a result of the teaching provided herein.

Without further description, it is believed that the skilled person can, using the preceding description, the following illustrative examples and the common general knowledge make use of the present invention. The following working examples point to some of the preferred embodiments of the present invention, and are not to be construed as limiting in any way on the remainder of the disclosure.

### Materials & Methods

### Cell lines and tissue culture

The human melanoma cell lines IRNE and 1359-Mel were kindly provided by G. Pawalec (University Tuebingen, Germany) and have recently been published (Lazaridou, M.F., et al., J Clin Med, 2020. 9(9)).These cell lines were maintained in Roswell Park Memorial Institute 1640 Medium (RPMI, Invitrogen^{™}) supplemented with 10 % (v/v) fetal bovine serum (FCS) (PAA, Pashing, Austria), 0.1 mM non-essential amino acids (Gibco^{®}, Dublin, Ireland), 2 mM L-glutamine (Lonza^{®}, Basel, Switzerland) and 1 % penicillin/streptomycin (v/v; PAA) at 37 °C, 5 % CO2. The HEK293T cell line was acquired from DSMZ and was cultured in DMEM medium (Sigma-Aldrich, Munich, Germany) supplemented with 10 % (v/v) FCS (PAA), 0.1 mM non-essential amino acids (Gibco), 2 mM L-glutamine (Lonza) and 1 % penicillin/streptomycin (v/v; PAA) at 37° C, 5 % CO2.

### Transfection and luciferase (luc) reporter gene assay

For miRNA mimic transfections, melanoma cells were seeded at 1×10⁵ cells/well in 6-well plates and were transfected with miRNA mimics or controls (both from Sigma^{®}, Kawasaki, Japan), respectively, at a final amount of 10 pM using Lipofectamine RNAi MAX (Invitrogen, Carlsbad, CA, USA) according to the manufacturers' reverse transfection protocol.

The following reverse transfection method was used to reverse transfect miRNA mimics into mammalian cells in a 24-well format. In reverse transfections, the complexes are prepared inside the wells, after which cells and medium are added. Reverse transfections are faster to perform than forward transfections, and are the method of choice for high-throughput transfection.

The transfection was optimized for the cell line used. All amounts and volumes are given on a per well basis.
1. For each well to be transfected, RNAi duplex-Lipofectamine RNAiMAX complexes were prepared as follows. 6 pmol RNAi duplex were diluted in 100 µl Opti-MEM I Medium without serum in the well of the tissue culture plate and gently mixed. Lipofectamine RNAiMAX was gently mixed before use, then 1 µl Lipofectamine RNAiMAX was added to each well containing the diluted RNAi molecules, gently mixed and incubated for 10-20 minutes at room temperature.
2. Cells were diluted in complete growth medium without antibiotics so that 500 µl contains the appropriate number of cells to give 30-50% confluence 24 hours after plating. 20,000-50,000 cells/well for suspension cells were used.
3. 500 µl of the diluted cells were added to each well with RNAi duplex - Lipofectamine RNAiMAX complexes. This gave a final volume of 600 µl and a final RNA concentration of 10 nM. The plates were mixed by gently rocking the plates back and forth.
4. The cells were incubated 24-72 hours at 37°C in a CO₂ incubator until ready to assay for gene knockdown.

Cells were harvested 48 hours after transfection and directly subjected to analysis.

For the luciferase (luc) reporter assay, 1×10⁴ HEK293T cells/well were seeded in 96-well plates. After 16 h, 10 ng of the reporter plasmid (pmirGLO Dual-Luciferase miRNA Target Expression Vector, Promega^{™}) in combination with the respective miRNA mimic or control (Sigma) at a final concentration of 25 nM were transfected using Lipofectamine^{™} 2000 (Invitrogen) according to the manufacturers' protocol. Luc activity was determined 48 h after transfection employing the Dual-Glo^{®} Luciferase substrate (Promega) according to the manufacturers' protocol. Luminescence was determined by a Tecan Infinite 200 Pro plate reader device. Relative luc activity was determined by normalizing the firefly reporter to renilla luc activity.

### RNA isolation, cDNA synthesis and quantitative PCR

Total RNA was isolated from cultured cells using the NucleoSpin^{®} miRNA kit (Macherey & Nagel) according to the manufacturers' instructions followed by DNase I treatment (NEB). RNA quality and quantity were assessed by spectrophotometric analysis and 500 ng of total RNA was used for cDNA synthesis (RevertAid H Minus First Strand cDNA synthesis kit, Fermentas, Waltham, MS, USA).

For miRNA-specific cDNA synthesis a miRNA-specific stem-loop primer was used, while reverse transcription of mRNAs required a random hexamer primer (Fermentas). Expression levels were analyzed by qRT-PCR using GoTaq^{®} qPCR Master Mix (Promega). For all primer pairs, an annealing temperature of 60°C was used. Relative changes of mRNA/miRNA amounts were determined by the ΔΔCt method using U6 snRNA, U44 snoRNA, U46 snoRNA and 47sno for normalization. All primers are listed in Table 3 below.

**Table 3. primers**

| **primer name** | **sequence** | **SEQ ID Nos** |
|---|---|---|
| miR-0009-5p qPCR FW | GCCCGCTCTTTGGTTATCTAGC | SEQ ID NO: 65 |
| miR-0009-5p SL RT | GTCGTATCCAGTGCAGGGTCCGAGGTATTCGCACTGGATAC- GAC TCATAC | SEQ ID NO: 66 |
| miR-0015a-5p qPCR FW | GCCCTAGCAGCACATAATGG | SEQ ID NO: 67 |
| miR-0015a-5p RT SL | GTCGTATCCAGTGCAGGGTCCGAGGTATTCGCACTGGATAC- GAC CACAAA | SEQ ID NO: 68 |
| miR-0016-5p qPCR FW | GCCCCTAGCAGCACGTAAATA | SEQ ID NO: 69 |
| miR-0016-5p SL RT | GTCGTATCCAGTGCAGGGTCCGAGGTATTCGCACTGGATAC- GAC CGCCAA | SEQ ID NO: 70 |
| miR-0017-5p qPCR FW | GCCCCAAAGTGCTTACAGTG | SEQ ID NO: 71 |
| miR-0017-5p RT SL | GTCGTATCCAGTGCAGGGTCCGAGGTATTCGCACTGGATAC- GAC CTACCT | SEQ ID NO: 72 |
| miR-0025-3p qPCR FW | GCCCCATTGCACTTGTCTCG | SEQ ID NO: 73 |
| miR-0025-3p RT SL | GTCGTATCCAGTGCAGGGTCCGAGGTATTCGCACTGGATAC- GAC TCAGAC | SEQ ID NO: 74 |
| miR-0026a RT SL | GTCGTATCCAGTGCAGGGTCCGAGGTATTCGCACTGGATAC- GAC AGCCTA | SEQ ID NO: 75 |
| miR-0026a-5p qPCR FW | GCCCTTCAAGTAATCCAGGA | SEQ ID NO: 76 |
| miR-0026b-5p SL RT | GTCGTATCCAGTGCAGGGTCCGAGGTATTCGCACTGGATAC- GAC ACCTAT | SEQ ID NO: 77 |
| miR-0027a-3p qPCR FW | CACGCATTCACAGTGGCTAAG | SEQ ID NO: 78 |
| miR-0027a-3p SL RT | GTCGTATCCAGTGCAGGGTCCGAGGTATTCGCACTGGATAC- GAC GCGGAA | SEQ ID NO: 79 |
| miR-0027b-3p qPCR FW | GCCCTTCACAGTGGCTAAGT | SEQ ID NO: 80 |
| miR-0027b-3p RT SL | GTCGTATCCAGTGCAGGGTCCGAGGTATTCGCACTGGATAC- GAC GCAGAA | SEQ ID NO: 81 |
| miR-0029a-3p qPCR FW | GCCCTAGCACCATCTGAAAT | SEQ ID NO: 82 |
| miR-0029a-3p RT SL | GTCGTATCCAGTGCAGGGTCCGAGGTATTCGCACTGGATAC- GAC TAACCG | SEQ ID NO: 83 |
| miR-0029b qPCR FW | GCCCTAGCACCATTTGAAATC | SEQ ID NO: 84 |
| miR-0029b RT SL | GTCGTATCCAGTGCAGGGTCCGAGGTATTCGCACTGGATAC- GAC AACACT | SEQ ID NO: 85 |
| miR-0034a qPCR FW | GCCCCAATCAGCAAGTATACTGC | SEQ ID NO: 86 |
| miR-0034a RT SL | GTCGTATCCAGTGCAGGGTCCGAGGTATTCGCACTGGATAC- GAC AGGGCA | SEQ ID NO: 87 |
| miR-0034a-5p qPCR FW | GCCCTGGCAGTGTCTTAG | SEQ ID NO: 88 |
| miR-0034a-5p SL RT | GTCGTATCCAGTGCAGGGTCCGAGGTATTCGCACTGGATAC- GAC ACAACC | SEQ ID NO: 89 |
| miR-0103a-3p qPCR FW | GCCCAGCAGCATTGTACAGG | SEQ ID NO: 90 |
| miR-0103a-3p RT SL | GTCGTATCCAGTGCAGGGTCCGAGGTATTCGCACTGGATAC- GAC TCATAG | SEQ ID NO: 91 |
| miR-0103b-5p qPCR FW | GCCCTCATAGCCCTGTACAA | SEQ ID NO: 92 |
| miR-0103b-5p RT SL | GTCGTATCCAGTGCAGGGTCCGAGGTATTCGCACTGGATAC- GAC AGCAGC | SEQ ID NO: 93 |
| miR-0146a-5p qPCR FW | CACGCATGAGAACTGAATTCC | SEQ ID NO: 94 |
| miR-0146a-5p SL RT | GTCGTATCCAGTGCAGGGTCCGAGGTATTCGCACTGGATAC- GAC AACCCA | SEQ ID NO: 95 |
| miR-0148a-3p qPCR FW | GCCCTCAGTGCACTACAGAAC | SEQ ID NO: 96 |
| miR-0148a/b-3p RT SL | GTCGTATCCAGTGCAGGGTCCGAGGTATTCGCACTGGATAC- GAC ACAAAG | SEQ ID NO: 97 |
| miR-0148b-3p qPCR FW | GCCCTCAGTGCATCACAGAAC | SEQ ID NO: 98 |
| miR-0152-3p qPCR FW | GCCCTCAGTGCATGACAGA | SEQ ID NO: 99 |
| miR-0152-3p RT SL | GTCGTATCCAGTGCAGGGTCCGAGGTATTCGCACTGGATAC- GAC CCAAGT | SEQ ID NO: 100 |
| miR-0155-5p qPCR FW | CACGCATTAATGCTAATCGTGAT | SEQ ID NO: 101 |
| miR-0155-5p SL RT | TCGTATCCAGTGCAGGGTCCGAGGTATTCGCACTGGATAC- GAC ACCCCT | SEQ ID NO: 102 |
| miR-0181a-5p qPCR FW | CACGCAAACATTCAACGCTGTC | SEQ ID NO: 103 |
| miR-0181a-5p SL RT | TCGTATCCAGTGCAGGGTCCGAGGTATTCGCACTGGATAC- GAC ACTCAC | SEQ ID NO: 104 |
| miR-0181b-5p qPCR FW | GCCCAACATTCATTGCTGTC | SEQ ID NO: 105 |
| miR-0181b-5p RT SL | GTCGTATCCAGTGCAGGGTCCGAGGTATTCGCACTGGATAC- GAC ACCCAC | SEQ ID NO: 106 |
| miR-0186-5p qPCR FW | GCCCCAAAGAATTCTCCTTT | SEQ ID NO: 107 |
| miR-0186-5p RT SL | GTCGTATCCAGTGCAGGGTCCGAGGTATTCGCACTGGATAC- GAC AGCCCA | SEQ ID NO: 108 |
| miR-0200c-3p qPCR FW | GCCCTAATACTGCCGGGTAA | SEQ ID NO: 109 |
| miR-0200c-3p RT SL | GTCGTATCCAGTGCAGGGTCCGAGGTATTCGCACTGGATAC- GAC TCCATC | SEQ ID NO: 110 |
| miR-0320a SL RT | GTCGTATCCAGTGCAGGGTCCGAGGTATTCGCACTGGATAC- GAC TCGCCC | SEQ ID NO: 111 |
| miR-0320a/b qPCR FW | GCCAAAAGCTGGGTTGAGAG | SEQ ID NO: 112 |
| miR-0324-5p qPCR FW | GAAACGCATCCCCTAGGGCAT | SEQ ID NO: 113 |
| miR-0324-5p RT SL | GTCGTATCCAGTGCAGGGTCCGAGGTATTCGCACTGGATAC- GAC ACACCA | SEQ ID NO: 114 |
| miR-0340-5p qPCR FW | GCCCGGTTATAAAGCAATGAGAC | SEQ ID NO: 115 |
| miR-0340-5p RT SL | GTCGTATCCAGTGCAGGGTCCGAGGTATTCGCACTGGATAC- GAC AATCAG | SEQ ID NO: 116 |
| miR-0424-5p qPCR FW | CACGCACAGCAGCAATTCATG | SEQ ID NO: 117 |
| miR-0424-5p SL RT | TCGTATCCAGTGCAGGGTCCGAGGTATTCGCACTGGATAC- GAC TTCAAA | SEQ ID NO: 118 |
| miR-0516b-5p qPCR FW | GCGCAATCTGGAGGTAAGAAGC | SEQ ID NO: 119 |
| miR-0516b-5p SL RT | GTCGTATCCAGTGCAGGGTCCGAGGTATTCGCACTGGATAC- GAC AAAGTG | SEQ ID NO: 120 |
| SL qPCR RV | CCAGTGCAGGGTCCGAGGTA | SEQ ID NO: 121 |
| U6 snRNA qPCR FW | CGGCAGCACATATACTAAAATTGGA | SEQ ID NO: 122 |
| U6 snRNA qPCR RV | AATATGGAACGCTTCACGAATTTGC | SEQ ID NO: 123 |
| RNU44 FW | CCTGGATGATGATAAGCAAATGC | SEQ ID NO: 124 |
| RNU44 RV | CAGTTAGAGCTAATTAAGACCTTC | SEQ ID NO: 125 |
| RNU46 FW | GGGTGATGAAAAAGAATCCTTAGG | SEQ ID NO: 126 |
| RNU46 RV | GTGTAACTATGACAAGTCCTTGC | SEQ ID NO: 127 |
| RNU47 FW | GATGTAATGATTCTGCCAAATG | SEQ ID NO: 128 |
| RNU47 RV | CCTCAGAATCAAAATGGAACG | SEQ ID NO: 129 |

### Flow cytometry

For flow cytometric analysis the following monoclonal antibodies (mAbs) were employed: APC conjugated PD-L1 (clone MIH3, Invitrogen) and FITC conjugated annexin V (Miltenyl Biotech). Cells were stained with the annexin V mAb as recommended by the manufacturers and then stained with the anti-PD-L1 mAb at a 1:100 dilution for 30 min at 4°C. The measurements were performed on a BD LSR Fortessa unit (Becton Dickinson, Heidelberg, Germany). FlowJo software (FlowJo, LLC) was used for analyses and to display flow cytometry results. The results are presented in histograms as mean fluorescence intensity (MFI).

### Western blot analysis

For the quantification of PD-L1 protein expression, total protein from transfected and control cells was extracted and quantified using the Pierce BCA protein assay kit (Thermo Fisher, Waltham, MS, USA). 50 µg of total protein was loaded on 10% denaturing polyacrylamide gels and transferred onto nitrocellulose membranes (GE Healthcare, Chicago, IL, USA). The membranes were blocked in TBS-T, 5%(w/v) skimmed milk for 1h and incubated with the primary antibodies directed against PD-L1 (CAL10 clone, abcam, 1:1000 dilution in TBS-T, 5%(w/v) bovine serum albumin (BSA)) and β-actin (clone, abcam, Cambridge, UK, 1:2000 dilution in TBS-T, 5% (w/v) BSA) overnight at 4°C. For detection, a secondary antibody conjugated with horseradish peroxidase (HRP) and the Lumi-Light substrate (Roche Applied Science) were applied. The signal was recorded with a LAS3000 camera system (Fuji LAS3000, Fuji GmbH, Düsseldorf) using the Image Reader LAS3000 software. The immunostaining signals were subsequently analyzed using the ImageJ software (NIH, Be-thesda, MD, USA). Quantification was done by setting the peak values of the control protein (β-actin) for each loaded sample as "1".

### miRNA enrichment assay (miTRAP) and small RNA sequencing

To identify potential miRNAs targeting the 3'-UTR or the CDS of PD-L1, the miTRAP method was employed, as recently described in Friedrich, M. et al., Journal for immunotherapy of cancer 8.1 (2020) and based on Braun, J, et al., Nucleic acids research 42.8 (2014): e66-e66 each of which is incorporated herein in their entireties. The miTRAP eluates were either used for cDNA synthesis of candidate or control miRNAs or subjected to small RNA sequencing (RNA-seq), which was carried out at Novogene (Hong-Kong, China) and analyzed as published (Friedrich, M. et al., Journal for immunotherapy of cancer 8.1 (2020).

### Statistical analysis

Mann-Whitney tests, chi-square tests and survival analysis were performed and plotted using GraphPad Prism v.8.0.1. Multivariate cox regression survival analysis was completed in IBM SPSS statistics 25. The data were considered significant with a p value < 0.05.

### Example 1: Identification of PD-L1 targeting miRNA candidates

PD-L1 regulating miRNAs were identified using the miTRAP method (Braun, J., et al., Nucleic Acids Res, 2014. 42(8): p. e66) (Figure 1A), followed by RNA-seq (Tretbar, U.S., et al., Methods Mol Biol, 2019. 1913: p. 81-101. As bait, *in vitro* transcribed RNA representing the CDS and the 3'-UTR sequence, which was split due to its large size into two parts (see Table 2, 3'UTR-1 and 3'UTR-2), were used. Since an important requirement for the outcome of the miTRAP analysis is the selection of a suitable cell line, the PD-L1 mRNA and protein expression profiles of 49 different melanoma cell lines were monitored regarding a discordant PD-L1 expression as indicator of a post-transcriptional regulation.

Based on these data the melanoma cell line IRNE was chosen for miTRAP analysis, since it showed a low surface expression of PD-L1 despite high mRNA levels, which is indicative of a posttranscriptional regulation of PD-L1. Following miRNA enrichment co-purified miRNAs were assessed by high-throughput small RNA-seq. Libraries were generated from the PD-L1 CDS, both parts of the 3'-UTR (3'UTR-1 and 3'UTR-2) and MS2 control miTRAP eluates. The RNA-seq results revealed high levels of co-precipitated miRNAs within the eluates of the different regions investigated (Figure 1B), with 142 unique miRNAs detected. The high reliability of this method was underlined by the fact that more than 75% of the known PD-L1 specific miRNAs were found using this approach (see Table in Figure 1F). 15 of the 142 detected miRNAs were equally found in the eluates of the PD-L1 CDS and both 3' UTRs. The highest number of individually identified miRNAs (n=62) were found in the eluates of the 3' UTR-1 as bait. After evaluation of the normalized read counts (TPM) and setting up stringent criteria for the identification of potential PD-L1-regulating miRNA candidates, such as (i) high normalized read counts and enrichment ratio, (ii) binding sites in at least 2 of the investigated PD-L1 sequences, (iii) multiple binding sites and (iv) binding prediction by at least three out of four applied tools, a number of miRNA candidates potentially regulating PD-L1 were selected. Their expression was validated by qPCR analysis of the miTRAP eluates as presented in Figure 1C-1E.

Of the confirmed miRNAs, 6 miRNAs, namely miR-26a-5p (SEQ ID NO: 1) miR-26b-5p (SEQ ID NO: 2), miR-29a-3p (SEQ ID NO: 3), miR-103b (SEQ ID NO: 4), miR-181b-5p (SEQ ID NO: 5) and miR-186-5p (SEQ ID NO: 6) were chosen to further investigate their contribution in PD-L1 regulation based on expression levels, number of predicted binding sites and immune modulatory functions in the literature. Based on its direct binding on PD-L1 mRNA as well as the regulation of c-Fos, a known transcription factor of PD-L1, miR-155-5p was included in the analysis despite absence of detection in the miTRAP eluates (low/no expression in the chosen IRNE cell line). In addition, miR-17-5p was also included as a positive control in the assays.

A selected summary of miRNA characteristics is provided in Table 4 below. A selected summary of the miRNA read counts obtained for the PD-L1 CDS and MS2 control are provided in Table 5.

| **Table 4**. Summary of miRNA characteristics | | | | | | |
|---|---|---|---|---|---|---|
| | **CDS** | | **3'-UTR-1** | | **3'-UTR-2** | |
| | Number of unique binding sites | seed region position | Number of unique binding sites | seed region position | Number of unique binding sites | seed re-gion position |
| **mir-17-5p** | 0 | | 0 | | 1 | 3437-3443 |
| **miR-29a-3p** | 0 | | 1 | 1557-1561 | 1 | 2986-2992 |
| **miR-103b** | 1 | 419-425 | 1 | 1977-1982 | 0 | |
| **miR-155-5p** | 0 | | 0 | | 2 | 2276-2282, 3528-3534 |
| **miR-181b-5p** | 0 | | 1 | 1625-1630 | 1 | 3576-3581 |
| **miR-186-5p** | 1 | 85-90 | 2 | 1454-1459, 1930-1935 | 3 | 2698-2683, 3011-3016, 3333-3339 |

**Table 5. Read counts for CDS, 3' UTR1 and 3'UTR-2 for selected miRNAs.**

| **miRNA** | **CDS** | **MS2 control** | **fold enrichment** |
|---|---|---|---|
| hsa-miR-584-5p | 9834.6 | 114.9 | 85.6 |
| hsa-miR-9-5p | 15423.9 | 206.9 | 74.6 |
| hsa-miR-550a-3-5p | 1429.3 | 23.0 | 62.2 |
| hsa-miR-550a-5p | 1429.3 | 23.0 | 62.2 |
| hsa-miR-454-3p | 1898.7 | 46.0 | 41.3 |
| hsa-miR-27b-3p | 69311.7 | 1792.8 | 38.7 |
| hsa-let-7g-5p | 14677.3 | 436.7 | 33.6 |
| hsa-miR-181a-5p | 6720.0 | 206.9 | 32.5 |
| hsa-miR-122-5p | 1685.3 | 69.0 | 24.4 |
| hsa-miR-3591-3p | 1685.3 | 69.0 | 24.4 |
| hsa-miR-20a-5p | 7829.3 | 321.8 | 24.3 |
| hsa-miR-27a-3p | 51626.4 | 2413.4 | 21.4 |
| hsa-miR-103a-3p | 23039.9 | 1172.2 | 19.7 |
| hsa-miR-185-3p | 1173.3 | 69.0 | 17.0 |
| hsa-miR-25-5p | 1514.7 | 91.9 | 16.5 |
| hsa-miR-222-3p | 18858.6 | 1402.1 | 13.5 |
| hsa-miR-629-5p | 4608.0 | 344.8 | 13.4 |
| hsa-miR-4521 | 8960.0 | 735.5 | 12.2 |
| hsa-let-7e-5p | 5781.3 | 482.7 | 12.0 |
| hsa-miR-211-5p | 2773.3 | 252.8 | 11.0 |
| hsa-miR-221-3p | 7829.3 | 758.5 | 10.3 |
| hsa-let-7f-5p | 10773.3 | 1264.2 | 8.5 |
| hsa-miR-128-3p | 3626.7 | 505.7 | 7.2 |
| hsa-miR-93-5p | 2538.7 | 367.8 | 6.9 |
| hsa-let-7i-5p | 10602.6 | 1563.0 | 6.8 |
| hsa-let-7c-5p | 3840.0 | 597.6 | 6.4 |
| hsa-miR-23a-3p | 1834.7 | 390.7 | 4.7 |
| hsa-miR-378a-3p | 4373.3 | 1011.3 | 4.3 |
| hsa-miR-105-5p | 1152.0 | 298.8 | 3.9 |
| hsa-let-7b-5p | 7040.0 | 2183.6 | 3.2 |
| hsa-miR-193a-5p | 4458.6 | 1448.1 | 3.1 |
| hsa-miR-451a | 1770.7 | 620.6 | 2.9 |
| hsa-miR-103a-2-5p | 1130.7 | 0.0 | 0 |
| hsa-miR-550b-3p | 1237.3 | 0.0 | 0 |

A selected summary of the miRNA read counts obtained for the PD-L1 3'UTR-1 and MS2 control are provided in Table 6.

**Table 6. Read counts for the 3' UTR1 for selected miRNAs.**

| **miRNA** | **3'UTR-1** | **MS2 control** | **fold enrichment** |
|---|---|---|---|
| hsa-miR-140-5p | 26822.2 | 207.7 | 129.1 |
| hsa-miR-425-5p | 6937.1 | 115.4 | 60.1 |
| hsa-miR-30b-5p | 1312.8 | 23.1 | 56.9 |
| hsa-miR-574-3p | 2125.8 | 92.3 | 23.0 |
| hsa-miR-29b-3p | 1759.3 | 92.3 | 19.1 |
| hsa-miR-9-5p | 3485.2 | 207.7 | 16.8 |
| hsa-miR-320b | 9669.3 | 692.4 | 14.0 |
| hsa-miR-25-3p | 6217.4 | 623.1 | 10.0 |
| hsa-miR-103a-3p | 11675.2 | 1177.0 | 9.9 |
| hsa-miR-320c | 1572.7 | 184.6 | 8.5 |
| hsa-miR-629-5p | 2419.0 | 346.2 | 7.0 |
| hsa-miR-24-3p | 17179.6 | 3208.0 | 5.4 |
| hsa-miR-3074-5p | 16853.0 | 3184.9 | 5.3 |
| hsa-miR-532-3p | 1186.2 | 230.8 | 5.1 |
| hsa-miR-181a-5p | 1039.6 | 207.7 | 5.0 |
| hsa-miR-27b-3p | 7223.7 | 1800.2 | 4.0 |
| hsa-miR-30c-5p | 5890.9 | 1546.3 | 3.8 |
| hsa-miR-151a-3p | 5977.5 | 1707.9 | 3.5 |
| hsa-miR-4521 | 2199.1 | 738.5 | 3.0 |
| hsa-miR-106b-3p | 1572.7 | 530.8 | 3.0 |
| hsa-miR-23a-3p | 1126.2 | 392.3 | 2.9 |

**Table 7. Read counts for the 3' UTR2 for selected miRNAs.**

| **miRNA** | **3'UTR-2** | **MS2** | **fold enrichment** |
|---|---|---|---|
| hsa-miR-20a-5p | 40453.9 | 94.7 | 427.4 |
| hsa-miR-146b-5p | 7683.5 | 74.4 | 103.3 |
| hsa-miR-3074-5p | 11949.7 | 933.1 | 12.8 |
| hsa-miR-24-3p | 12011.0 | 939.8 | 12.8 |
| hsa-miR-151a-3p | 4772.8 | 500.4 | 9.5 |
| hsa-miR-30e-5p | 1281.1 | 209.6 | 6.1 |
| hsa-miR-103a-3p | 1190.8 | 344.8 | 3.5 |
| hsa-miR-222-3p | 1394.1 | 412.5 | 3.4 |
| hsa-miR-340-5p | 364.7 | 108.2 | 3.4 |

**Table 8. List of known miRNA found in miTRAP eluates.**

| **known PD-L1 miRNAs (according to PMID: 31258527, 29186904)** | **found in our miTRAP eluates?** |
|---|---|
| miR-15a | yes |
| miR-15b | yes |
| miR-16 | yes |
| miR-17-5p | yes |
| miR-18a | yes |
| miR-33a | yes |
| miR-34a | yes |
| miR-93-5p | yes |
| miR-106-5p | yes |
| miR-138-5p | no |
| miR-140 | yes |
| miR-142-5p | no |
| miR-152 | yes |
| miR-155 | no |
| miR-193a-3p | no |
| miR-195 | yes |
| miR-200 | yes |
| miR-324-5p | yes |
| miR-338-5p | yes |
| miR-340 | yes |
| miR-383 | no |
| miR-424 | yes |
| miR-513 | no |
| **total** | **0.773** |

**Table 9. List of miRNA of the invention and target site location.**

| **miRNA** | **CDS** | **3' UTR-1** | **3' UTR-2** |
|---|---|---|---|
| hsa-miR-103a-3p | + | + | + |
| hsa-miR-103b | + | + | |
| hsa-miR-107 | + | + | + |
| miR186-5P | + | + | + |
| miR29a-3P | | + | + |
| miR-181b-5P | | + | + |
| hsa-miR-148a-3p | + | + | + |
| hsa-miR-148b-3p | + | + | + |
| hsa-miR-152-3p | + | + | + |
| hsa-miR-181a-5p | + | + | + |
| hsa-miR-181b-5p | + | + | + |
| hsa-miR-211-5p | + | + | + |
| hsa-miR-23a-3p | + | + | + |
| hsa-miR-25-5p | + | + | + |
| hsa-miR-34a-5p | + | + | + |
| hsa-miR-454-3p | + | + | + |
| hsa-miR-584-5p | + | + | + |
| hsa-miR-629-5p | + | + | + |
| hsa-miR-105-5p | + | | + |
| hsa-miR-17-5p | + | | + |
| hsa-miR-20a-5p | + | | + |
| hsa-miR-222-3p | + | | + |
| hsa-miR-451a | + | | + |
| hsa-miR-486-3p | + | | + |
| hsa-miR-486-5p | + | | + |
| hsa-miR-93-5p | + | | + |
| hsa-miR-185-3p | + | + | |
| hsa-miR-23b-3p | + | + | |
| hsa-miR-27a-3p | + | + | |
| hsa-miR-27a-5p | + | + | |
| hsa-miR-27b-3p | + | + | |
| hsa-miR-4521 | + | + | |
| hsa-miR-9-5p | + | + | |
| hsa-miR-135b-5p | | + | + |
| hsa-miR-140-5p | | + | + |
| hsa-miR-151a-3p | | + | + |
| hsa-miR-24-3p | | + | + |
| hsa-miR-26b-5p | | + | + |
| hsa-miR-27b-5p | | + | + |
| hsa-miR-3074-5p | | + | + |
| hsa-miR-340-5p | | + | + |
| hsa-miR-509-3p | | + | + |

### Example 2: Regulatory effect of selected miRNAs on PD-L1 protein level

In order to determine the possible regulatory effects of the identified miRNAs on PD-L1 expression *in vitro,* melanoma cells were transfected with selected miRNA mimics. The cell line 1359-Mel was chosen, due to its high transfection efficiency and moderate to high levels of both mRNA and protein PD-L1 expression to minimize possible preexisting post-transcriptional regulation. Downregulation of PD-L1 surface expression was found upon transfection with miRNA mimics, which ranged from about 10 to about 30% downregulation (Figure 2A). Using flow cytometry, transfection of miR-103b and miR-181b caused reduced PD-L1 expression between about 10% and about 20%, miR-186 overexpression downregulated PD-L1 expression up to about 25%. These results were confirmed by Western blot of transfectants demonstrating a reduced surface expression of PD-L1 (Figure 2B).

### Example 3: Assessment of miRNA binding on selected PD-L1 sequences

In order to confirm whether the observed downregulation of PD-L1 expression upon transfection with the candidate miRNAs is a direct effect of their binding to the PD-L1 3'-UTR or CDS dual luciferase (luc) assays were conducted. The assays were carried out for the miRNAs with *in silico* predicted binding sites and their respective PD-L1 regions (Figure 3A). miR-155-5P reduced the activity > 60% compared to the mock control. miR-181b lead to an equal signal reduction of approximately 30% in both 3'-UTR segments (Figure 3B). Furthermore, miR-103b transfectants demonstrated the second highest decrease of in luc activity of almost 50% targeting the CDS (Figure 3B). The biding site for multiple regions of selected miRNA of the invention is shown in Figure 3C.

In conclusion, it has been shown that the downregulation of PD-L1 by the miRNAs of the invention is a direct effect of the binding of the miRNAs to the CDS, 3'UTR-1 and/or 3'UTR-2 regions of the PD-L1 mRNA.

### Example 4: Generation of the PD-L1 miRNA (PDM) score

A combinatorial analyses were performed. Due to the fact that each of the analyzed 5 miRNAs caused different effects on the PD-L1 surface expression upon transfection, their impact on the amount of reduction was used to further stratify them. MiRNAs resulting in less than a 10 % reduction of PD-L1 expression in the mel1379 melanoma cells were attributed a score of 1 (+) (e.g. miR-29a), while miRNAs reducing the PD-L1 expression level up to 20 % were scored 2 (++) (e.g. miR-181b) and miRNAs reducing the PD-L1 expression level by > 20 % received a score (miRNA maximum point worth) of 3 (+++) (Figure 4A). Moreover, individual miRNA expression levels within the melanoma patients were separated into quartiles. Low miRNA expression levels (first quartile) were considered as weak contributors and scored with 0 points (Figure 4B), while miRNAs reaching an expression level within the higher quartiles were categorized with an additional miRNA score (maximum: 3) for each subsequent quartile (Figure 4B). As a result, each patient received five individual miRNA scores (miR-29, miR-155, miR-181b, miR-186, miR-17). The total sum of all individual miRNA scores for each patient represented the patients' PD-L1 miRNA score (PDM score) with possible values spanning between 0 and 12 (Figure 4D).

### Example 5: Correlation of the PDM score with the patients' clinicopathological characteristics

In order to further investigate the possible significance of the PDM score generated, arbitrary thresholds were set based on the miRNA expression patterns to separate patients in three distinct groups. The three groups were PDM_{low} (0<PDM_{low}≤3), PDMᵢₙₜ (3<PDMᵢₙₜ≤7) and PDM_{high} (7<PDM_{high}≤12), as indicated in Figure 4D. Patients with a high CD8+ T cell infiltration had significantly higher PDM scores (Figure 7B). Survival analyses demonstrated that patients with an intermediate to high PDM score had a significantly lower disease-free survival (DFS) than patients in the PDM_{low} group (Figure 5A). In particular, patients with a PDM score > 3 (PDMᵢₙₜ, PDM_{high}) had a 4.4 -5.2 times higher risk to relapse compared to patients with a PDM score < 3 (Figure 5B). However, it is noteworthy that the PDM score was not linked in this analysis to the PD-L1 expression. Multivariate survival analysis not only demonstrated a higher significance of the PDM score groups compared to N status and ulceration (Figure 5C), but also represented an independent prognostic factor together with the T status for patients' DFS (Figure 5C) in our patient cohort.

### Example 6: Prognostic value of the PDM score by combining PD-L1 expression and immune infiltration

Since the immune infiltrate might contribute to the PD-L1 expression as well as the miRNA profile, it was determined whether the PDM scoring system was dependent on a combination of the PD-L1 positivity (PD-L1⁺), as determined by immunohistochemistry, and the frequency of T cell infiltration. Patients' DFS varied in the PDM_{high} and PDMᵢₙₜ score groups and was inversely associated with the PD-L1 surface expression. Despite a high PDM score, the PD-L1 positivity (PD-L1⁺) was accompanied by a lower DFS (Figure 6A). The opposite was found for the PDMᵢₙₜ group. In addition, the immune cell infiltration was analyzed in the different PDM patients' groups. In PDMᵢₙₜ patients, but not in the other two groups, the surface expression of PD-L1 was accompanied by a high CD8+ and CD4+ T cell infiltration, (Figure 8). Based on this information, a combined signature (comPDM) was generated that simultaneously utilized the PDM score, the PD-L1 positivity (PD-L1⁺) and CD8+ T cell infiltration in the tumor tissue. The comPDM₁ group consisted of the PDM_{low} patients regardless of PD-L1 positivity and CD8+ T cell infiltration. The medium prognostic group, termed comPDM₂, consisted of PDM_{high}/PD-L1⁻ patients and PDMᵢₙₜ/CD8+high (Figure 6B), while the comPDM₃ group consisted of PDM_{high}/PD-L1⁺ and PDMᵢₙₜCD8+low patients. Thus, this combination identified a signature (comPDM), which was able to stratify the melanoma patients into three groups with distinct prognosis (Figure 6B). Finally, multivariate analysis suggested that the comPDM signature is an independent prognostic factor with a prognostic value comparable to the T status and a hazard ratio of 3.579 between the comPDM groups (Figure 6C) in the cohort of analyzed patients.

### Example 7: Expression of PD-L1 regulating miRNA candidates on melanoma samples

Formalin-Fixed Paraffin-Embedded (FFPE) samples from 46 melanoma patients were collected at the University Hospital of Zurich, Switzerland. The clinicopathological characteristics of the patients are summarized in Figure 5B. Evaluation of miRNA expression highlighted that miR-29a (SEQ ID NO: 3), miR-181b (SEQ ID NO: 5) and miR-17-5P (SEQ ID NO: 8) had consistently higher expression levels in all melanoma samples in comparison to miR-155-5P (SEQ ID NO: 7) and miR-186-5P (SEQ ID NO: 6), the miRNAs (Figure 5A). By comparing the miRNA expression pattern with PD-L1 positivity, a slightly lower expression of miR-29a (SEQ ID NO: 3) and miR-181b (SEQ ID NO: 5) was detected on PD-L1⁺ melanoma samples (Figure 5A). In order to determine a possible differential regulation of the miRNAs between PD-L1⁻ and PD-L1⁺ patients, a correlation matrix was generated.

Despite some correlations were found in all patients regardless of their PD-L1 status, PD-L1⁺ melanoma lesions showed a stronger correlation between miR-186 and miR-155/miR-181b, while miR-29a and miR-17 were more strongly correlated in PD-L1⁻ patients. Some correlations unique to PD-L1⁺ lesions, such as e.g. miR-181b-5P, appeared to have strong association with all investigated miRNAs. These results therefore show that multiple miRNAs of the invention are related by common nodes of regulation. The simultaneous expression or overexpression of miRNAs belonging to same or different nodes of regulation is therefore expected to provide advantageous effects.

In conclusion, the differential correlations observed are an indication of an altered microenvironment on the PD-L1⁺ lesions, represented by immune subpopulations expressing the miRNAs of interest, or more importantly suggest the contribution of these miRNAs in a common pathway, which indirectly supports PD-L1 mediated immune escape through our targets of interest.

### Discussion

The created PDM scoring system based on the functional efficiency of the identified miRNAs on the PD-L1 surface expression have a very strong prognostic value in our cohort of melanoma patients. The PDM score is a negative independent prognostic factor along with the T status regardless of other clinicopathological characteristics. These data indicate that its prognostic nature cannot be solely attributed to the PD-L1 regulation, but could be connected to additional targets of our miRNA candidates.

The prognostic value of the suggested comPDM signature was increased by considering the PDM score, the PD-L1 expression level as well as the T cell infiltrate in the melanoma lesions. The comPDM signature stratifies our patients in three distinct groups. In the first group the low PDM score, indicating the reduced expression of our miRNA candidates, allows for increased DFS. As the PDM score increases, patients with intermediate PDM score, but high enough CD8+ T cell infiltration (accompanied with PD-L1 expression), as well as PDM_{high} patients whose high miRNA levels appear to actively suppress CD274 have slightly worse prognosis. Lastly, the protecting nature of CD8+ T cells appears insufficient within PDM_{high}/PD-L1+ patients. As high expression levels of the miRNAs s identified have been shown to correlate with a worse prognosis, the importance of CD8+ T cell infiltration is underlined in melanoma lesions, which might benefit from the miRNA-induced increased proliferation. Thus, poorly infiltrated tumors do not necessarily require high PD-L1 expression for immune escape. This might be explained next to the general negative impact of the PDM score, by the fact that the high expressed miRNAs could indicate the presence of non-effector CD8+ T cell subtypes, as miRNA expression differs throughout T cell maturation. As a result, these patients together with the PDMᵢₙₜ patients without CD8+ T cell infiltration were placed in the comPDM₃ group, which has the worst prognosis within our cohort. These data suggest an interplay between miRNAs, PD-L1 and the immune infiltrate, which might allow us to better optimize potential miRNA therapies, to be used as a single therapy or in combination with CPI. The involvement of these miRNAs in the regulation of various pro-and anti-oncogenic pathways in both immune and tumor cells indicate that the generation of combinatorial functional signatures is essential not only for improved prognosis, but also for the assessment of therapeutic options with the minimum amount of drawbacks for the patient.

Even though the invention has been disclosed with reference to specific embodiments, it is apparent that other embodiments and variations of this invention may be devised by the

## Claims

1. A method for prognosing a cancer that expresses PD-L1 on the surface of tumor cells (PD-L1-positive cancer) in a patient, the method comprising the steps of:
a) measuring in a sample from the patient the tumor expression level of at least one miRNA selected from the group consisting of the miRNAs according to SEQ ID NO: 1 to 60;
b) comparing the tumor expression level determined in step a) with the tumor expression level of the same at least one miRNA in a reference patient group suffering from the same cancer, to determine a relative tumor expression level of the at least one miRNA; and
c) determining a prognosis for the cancer based on the outcome of step b), wherein a high relative tumor expression level of the at least one miRNA relative to the expression of the at least one miRNA in the reference group contributes to a poor prognosis.

2. The method of claim 1, wherein the tumor expression level of one, two, three, four or five of the miRNAs selected from the group consisting of miR-29, miR-155, miR-181b, miR-186 and miR-17 is determined, preferably the tumor expression level of miR-29, miR-155, miR-181b, miR-186 and miR-17 is determined.

3. The method of any one of the preceding claims, wherein the method further comprises
the step of establishing a cell culture of a cell line that expresses PD-L1 on its surface and the step of measuring in vitro the reduction of PD-L1 surface expression of the cell line that expresses PD-L1 on its surface upon administration of the at least one miRNA or a miRNA mimic thereof; and
wherein step c) is further based on the outcome of the step of the measuring in vitro the reduction of PD-L1 surface expression.

4. The method of any one of the preceding claims, wherein the method further comprises
a step of measuring in a sample from the patient PD-L1 surface expression on the tumor cells; and
wherein step c) is further based on the outcome of the step of measuring in a sample from the patient PD-L1 surface expression.

5. The method of any one of the preceding claims, wherein the method further comprises
a step of measuring in a sample from the patient CD8+ T cell infiltration in the tumor tissue; and
wherein step c) is further based on the outcome of the further step of measuring in a sample from the patient CD8+ T cell infiltration in a tumor tissue of the PD-L1-positive cancer.

6. The method of any one of the preceding claims, wherein the cancer is selected from the group consisting of skin cancer, preferably melanoma, non-small cell lung cancer (NSCLC), Hodgkin's lymphoma, bladder cancer, renal cell carcinoma (RCC), head and neck squamous cell carcinoma (HNSCC), breast cancer, Merkel cell carcinoma, hepatocellular carcinoma (HCC) and gastric cancer (GC).

7. The method of any one of the preceding claims, wherein a numerical value (miRNA worth) is attributed to each selected miRNA, wherein the miRNA worth corresponds to the degree of reduction that the miRNA or a miRNA mimic thereof causes in PD-L1 surface expression upon administration of the miRNA or a miRNA mimic thereof in cell culture to a PD-L1-positive cancer cell line, wherein the greater the reduction in PD-L1 surface expression upon administration of the miRNA or a miRNA mimic thereof, the greater the miRNA worth, wherein the miRNA worth is a positive number.

8. The method of any one of the preceding claims, wherein a numerical value (expression multiplier) is attributed to each selected miRNA, wherein the expression multiplier corresponds to the tumor expression level of the miRNA as compared to the tumor expression level of the same miRNA in a reference group of patients suffering from the same cancer, wherein the greater the expression of the miRNA in the patient relative to the reference group of patients, the greater the expression multiplier, wherein the expression multiplier is a positive number or zero.

9. The method of claim 8, wherein the method further comprises calculating a numerical individual miRNA score for each selected miRNA that is the product of the miRNA worth for the selected miRNA and the expression multiplier (miRNA worth*expression multiplier) for the same selected miRNA.

10. The method of claim 9, wherein the method further comprises calculating a numerical PDM score, which PDM score is the sum of individual miRNA scores, by addition of each individual miRNA score for each selected miRNA, wherein the greater the PDM score, the poorer the prognosis.

11. The method of claim 10, wherein the miRNA worth for miR-29 is 1*a, the miRNA worth for miR-155 is 3*a, the miRNA worth for miR-181b is 2*a, the miRNA worth for miR-186 is 3*a and the miRNA worth for miR-17 is 3*a, wherein a is a factor and a positive number, preferably 1.

12. Use of at least one miRNA selected from the group consisting of the miRNAs according to SEQ ID NO: 1 to 60 for prognosing PD-L1 positive cancer.

13. The use of claim 11, wherein one, two, three, four or five miRNAs selected from the group consisting of miR-29, miR-155, miR-181b, miR-186 and miR-17 are used for prognosing PD-L1 positive cancer.

14. A kit for prognosing PD-L1 positive cancer, the kit comprising polymerase chain reaction primers or a microarray capable of detecting the expression of at least one miRNA selected from the group consisting of the miRNAs according to SEQ ID NO: 1 to 60.

15. The kit of claim 13, wherein the polymerase chain reaction primers or the microarray are capable of detecting the expression of one, two, three, four or five miRNAs selected from the group consisting of miR-29, miR-155, miR-181b, miR-186 and miR-17.
